# EUROPÄISCHE PATENTSCHRIFT

(11) **EP 1 414 850 B1**
(45) Veröffentlichungstag und Bekanntmachung des Hinweises auf die Patenterteilung: **19.06.2013**
(21) Anmeldenummer: 02767329.2
(22) Anmeldetag: 06.08.2002
(51) Int. Cl.: C07K 14/705, G01N 33/68

(54) **GENETISCH MODIFIZIERTE ZYKLISCH-NUKLEOTID-GESTEUERTE IONENKANÄLE UND DEREN VERWENDUNG**
GENETICALLY MODIFIED CYCLIC-NUCLEOTIDE CONTROLLED ION CHANNELS AND THE USE THEREOF
CANAUX IONIQUES GENETIQUEMENT MODIFIES A COMMANDE NUCLEOTIDIQUE CYCLIQUE ET UTILISATION

(30) Priorität: 08.08.2001 DE 10138876
(43) Veröffentlichungstag der Anmeldung: 06.05.2004
(73) Patentinhaber: Forschungszentrum Jülich GmbH, 52425 Jülich (DE)
(72) Erfinder: KAUPP, Ulrich, B., 52062 Aachen (DE); SEIFERT, Reinhardt, 52428 Jülich (DE); GAUSS, Renate, 65929 Frankfurt (DE); KÖRSCHEN, Heinz-Gerd, 47804 Krefeld (DE)
(74) Vertreter: Fitzner, Ulrich
(86) Internationale Anmeldenummer: PCT/EP2002/008756
(87) Internationale Veröffentlichungsnummer: WO 2003/014149

(56) Entgegenhaltungen:
- WO-A-99/42574
- ALTENHOFEN W ET AL: "CONTROL OF LIGAND SPECIFICITY IN CYCLIC NUCLEOTIDE-GATED CHANNELS FROM ROD PHOTORECEPTORS AND OLFACTORY EPITHELIUM" PROCEEDINGS OF THE NATIONAL ACADEMY OF SCIENCES OF THE UNITED STATES, Bd. 88, Nr. 21, 1991, Seiten 9868-9872, XP002248595 1991 ISSN: 0027-8424 in der Anmeldung erwähnt
- SHABB J B ET AL: "MUTATING PROTEIN KINASE CAMP-BINDING SITES INTO CGMP-BINDING SITES MECHANISM OF CGMP SELECTIVITY" JOURNAL OF BIOLOGICAL CHEMISTRY, Bd. 266, Nr. 36, 1991, Seiten 24320-24326, XP002248596 ISSN: 0021-9258
- KAUPP U. B.: "THE CYCLIC NUCLEOTIDE-GATED CHANNELS OF VERTEBRATE PHOTORECEPTORS AND OLFACTORY EPITHELIUM" TRENDS IN NEUROSCIENCES, Bd. 14, Nr. 4, 1991, Seiten 150-157, XP008019924
- LUDWIG J ET AL: "PRIMARY STRUCTURE OF CYCLIC AMP-GATED CHANNEL FROM BOVINE OLFACTORY EPITHELIUM" FEBS LETTERS, ELSEVIER SCIENCE PUBLISHERS, AMSTERDAM, NL, Bd. 270, Nr. 1-2, 1990, Seiten 24-29, XP002205518 ISSN: 0014-5793
- KAUPP U B ET AL: "CYCLIC NUCLEOTIDE-GATED ION CHANNELS" PHYSIOLOGICAL REVIEWS, AMERICAN PHYSIOLOGICAL SOCIETY, US, Bd. 82, Nr. 3, Juli 2002 (2002-07), Seiten 769-824, XP001096371 ISSN: 0031-9333
- VARNUM M D ET AL: "Molecular mechanism for ligand discrimination of cyclic nucleotide-gated channels.", NEURON SEP 1995 LNKD- PUBMED:7546741, vol. 15, no. 3, September 1995 (1995-09), pages 619-625, ISSN: 0896-6273

### SEQUENZPROTOKOLL

<110> Forschungszentrum Jülich
<120> Genetisch modifizierte zyklisch-Nukleotid-gesteuerte Ionenkanäle und deren Verwendung
<130> Anmeldung FZJ-0102
<140>
   <141>
<160> 12
<170> PatentIn Ver. 2.1
<210> 1
   <211> 1989
   <212> DNA
   <213> Rind
<220>
   <221> CDS
   <222> (1) .. (1989)
   <223> Nukleinsäuresequenz der α3-Untereinheit des CNG-Kanals aus Rind
<400> 1
   atg aca gaa aaa gcc aat ggc gtg aag agc tcc cca gcc aat aac cac    48
   Met Thr Glu Lys Ala Asn Gly Val Lys Ser Ser Pro Ala Asn Asn His
     1               5                  10                  15
   aac cac cat gcc cct cct gcc atc aag gcc agt ggc aaa gat gac cac    96
   Asn His His Ala Pro Pro Ala Ile Lys Ala Ser Gly Lys Asp Asp His
                20                  25                  30
   agg gcc agc agc cgg cca cag tct gct gct gct gat gac acc tcc tca   144
   Arg Ala Ser Ser Arg Pro Gln Ser Ala Ala Ala Asp Asp Thr Ser Ser
            35                  40                  45
   gag cta cag caa ctg gca gag atg gat gcc ccc cag cag agg agg ggt   192
   Glu Leu Gln Gln Leu Ala Glu Met Asp Ala Pro Gln Gln Arg Arg Gly
   50 55 60
   ggc ttc cgc agg att gcc cgc ctg gtg ggg gtc ctc aga gag tgg gct 240
   Gly Phe Arg Arg Ile Ala Arg Leu Val Gly Val Leu Arg Glu Trp Ala
   65 70 75 80
   tac agg aac ttc cgt gag gag gag cct aga cct gac tca ttc ctt gag 288
   Tyr Arg Asn Phe Arg Glu Glu Glu Pro Arg Pro Asp Ser Phe Leu Glu
   85 90 95
   cgt ttc cgg ggg cct gag ctc cac acc gtg aca aca caa caa gga gac 336
   Arg Phe Arg Gly Pro Glu Leu His Thr Val Thr Thr Gln Gln Gly Asp
   100 105 110
   ggc aaa ggc gac aag gac ggc gag ggc aag ggc acc aag aag aag ttt 384
   Gly Lys Gly Asp Lys Asp Gly Glu Gly Lys Gly Thr Lys Lys Lys Phe
   115 120 125
   gaa ctc ttt gtc ttg gac cca gcc ggg gac tgg tac tac cgc tgg ctt 432
   Glu Leu Phe Val Leu Asp Pro Ala Gly Asp Trp Tyr Tyr Arg Trp Leu
   130 135 140
   ttt ctc att gcc ttg ccc gtc ctc tac aac tgg tgc cta ttg gtg gcc 480
   Phe Leu Ile Ala Leu Pro Val Leu Tyr Asn Trp Cys Leu Leu Val Ala
   145 150 155 160
   aga gcc tgc ttc agt gac ctg cag aaa ggc tac tac ata gtg tgg ctg 528
   Arg Ala Cys Phe Ser Asp Leu Gln Lys Gly Tyr Tyr Ile Val Trp Leu
   165 170 175
   gtg ctg gat tac gtc tca gat gtg gtc tac atc gca gac ctc ttc atc 576
   Val Leu Asp Tyr Val Ser Asp Val Val Tyr Ile Ala Asp Leu Phe Ile
   180 185 190
   cga ctg cgc aca ggt ttc ttg gag cag ggg cta ctg gtg aaa gac acc 624
   Arg Leu Arg Thr Gly Phe Leu Glu Gln Gly Leu Leu Val Lys Asp Thr
   195 200 205
   aag aag ttg cgg gac aac tac atc cac acc atg cag ttt aag ctg gat 672
   Lys Lys Leu Arg Asp Asn Tyr Ile His Thr Met Gln Phe Lys Leu Asp
   210 215 220
   gtg gcc tcc atc atc cct aca gac ctg atc tat ttt gct gtg ggg atc 720
   Val Ala Ser Ile Ile Pro Thr Asp Leu Ile Tyr Phe Ala Val Gly Ile
   225                 230                 235                 240
   cat aac cct gag gtg cgc ttc aac cgc ctg cta cac ttt gcc cgc atg    768
   His Asn Pro Glu Val Arg Phe Asn Arg Leu Leu His Phe Ala Arg Met
                   245                 250                 255
   ttt gag ttc ttt gac cgc act gag aca cgc acc agc tac ccc aac atc    816
   Phe Glu Phe Phe Asp Arg Thr Glu Thr Arg Thr Ser Tyr Pro Asn Ile
               260                 265                 270
   ttc cga ata agc aac ctg atc ctc tac atc ttg atc atc att cac tgg    864
   Phe Arg Ile Ser Asn Leu Ile Leu Tyr Ile Leu Ile Ile Ile His Trp
           275                 280                 285
   aat gcc tgc atc tac tat gcc atc tcc aag tcc atc ggc ttt ggg gta    912
   Asn Ala Cys Ile Tyr Tyr Ala Ile Ser Lys Ser Ile Gly Phe Gly Val
       290                 295                 300
   gac acc tgg gtt tac ccc aac atc act gac cct gag tat ggc tac ctg    960
   Asp Thr Trp Val Tyr Pro Asn Ile Thr Asp Pro Glu Tyr Gly Tyr Leu
   305                 310                 315                 320
   tct agg gag tac atc tat tgc ctt tac tgg tct aca ctg acc ctc acc    1008
   Ser Arg Glu Tyr Ile Tyr Cys Leu Tyr Trp Ser Thr Leu Thr Leu Thr
                   325                 330                 335
   acc att ggg gag aca cca ccc cct gta aag gat gag gag tac ctg ttt    1056
   Thr Ile Gly Glu Thr Pro Pro Pro Val Lys Asp Glu Glu Tyr Leu Phe
               340                 345                 350
   gtc atc ttt gac ttc ctg att ggt gtc ctc atc ttt gcc acc atc gtg    1104
   Val Ile Phe Asp Phe Leu Ile Gly Val Leu Ile Phe Ala Thr Ile Val
           355                 360                 365
   gga aat gtg ggc tcc atg atc tcc aac atg aat gcc acc cgg gct gag    1152
   Gly Asn Val Gly Ser Met Ile Ser Asn Met Asn Ala Thr Arg Ala Glu
       370                 375                 380
   ttc cag gcc aag att gat gct gtc aaa cat tat atg cag ttc cga aag    1200
   Phe Gln Ala Lys Ile Asp Ala Val Lys His Tyr Met Gln Phe Arg Lys
   385                 390                 395                 400
   gtc agc aag gag atg gaa gcc aag gtc att agg tgg ttt gac tac ttg   1248
   Val Ser Lys Glu Met Glu Ala Lys Val Ile Arg Trp Phe Asp Tyr Leu
                   405                 410                 415
   tgg acc aat aag aag agt gta gat gag cga gaa gtc ctc aaa aac ctg   1296
   Trp Thr Asn Lys Lys Ser Val Asp Glu Arg Glu Val Leu Lys Asn Leu
               420                 425                 430
   cca gca aag ctc agg gct gag ata gcc atc aac gtc cac ctg tcc aca   1344
   Pro Ala Lys Leu Arg Ala Glu Ile Ala Ile Asn Val His Leu Ser Thr
           435                 440                 445
   ctc aag aaa gtg cgc atc ttt cag gac tgt gag gct ggc ctg ctg gtg   1392
   Leu Lys Lys Val Arg Ile Phe Gln Asp Cys Glu Ala Gly Leu Leu Val
       450                 455                 460
   gaa ctg gta tta aag ctc cgg cct cag gtc ttt agc cct ggg gac tac   1440
   Glu Leu Val Leu Lys Leu Arg Pro Gln Val Phe Ser Pro Gly Asp Tyr
   465                 470                 475                 480
   att tgc cgc aag ggg gat att ggg aag gag atg tac ata atc aag gag   1488
   Ile Cys Arg Lys Gly Asp Ile Gly Lys Glu Met Tyr Ile Ile Lys Glu
                   485                 490                 495
   gga aaa ttg gca gtg gtg gct gat gac ggt gtc act cag tat gcc ctg   1536
   Gly Lys Leu Ala Val Val Ala Asp Asp Gly Val Thr Gln Tyr Ala Leu
               500                 505                 510
   ctc tcg gct ggg agt tgc ttt gga gag atc agt atc ctt aat att aag   1584
   Leu Ser Ala Gly Ser Cys Phe Gly Glu Ile Ser Ile Leu Asn Ile Lys
           515                 520                 525
   ggc agc aaa atg ggc aat cgg cgc aca gcc aac atc cgc agt ctt ggc   1632
   Gly Ser Lys Met Gly Asn Arg Arg Thr Ala Asn Ile Arg Ser Leu Gly
       530                 535                 540
   tac tct gat ctg ttc tgc ttg tcc aag gat gat ctt atg gaa gct gtg   1680
   Tyr Ser Asp Leu Phe Cys Leu Ser Lys Asp Asp Leu Met Glu Ala Val
   545                 550                 555                 560
   act gag tac cct gat gcc aag agg gtc ttg gag gag aga ggc cgg gag   1728
   Thr Glu Tyr Pro Asp Ala Lys Arg Val Leu Glu Glu Arg Gly Arg Glu
                   565                 570                 575
   att ctg atg aag gag ggc ttg ttg gat gag aat gag gtg gca gcc agc    1776
   Ile Leu Met Lys Glu Gly Leu Leu Asp Glu Asn Glu Val Ala Ala Ser
               580                 585                 590
   atg gag gta gat gtg cag gaa aag cta gaa cag ctg gag acc aac atg    1824
   Met Glu Val Asp Val Gln Glu Lys Leu Glu Gln Leu Glu Thr Asn Met
           595                 600                 605
   gac acc ttg tac act cgt ttt gcc cgc ctg ctg gcc gag tac acg gga    1872
   Asp Thr Leu Tyr Thr Arg Phe Ala Arg Leu Leu Ala Glu Tyr Thr Gly
       610                 615                 620
   gcc cag cag aag ctc aag cag cgc atc aca gtt ttg gaa acg aag atg    1920
   Ala Gln Gln Lys Leu Lys Gln Arg Ile Thr Val Leu Glu Thr Lys Met
   625                 630                 635                 640
   aag cag aat aat gag gat gac tcc ctg tca gat ggg atg aac agc cca    1968
   Lys Gln Asn Asn Glu Asp Asp Ser Leu Ser Asp Gly Met Asn Ser Pro
                   645                 650                 655
   gag cca cct gcc gag aag cca                                        1989
   Glu Pro Pro Ala Glu Lys Pro
               660
<210> 2
   <211> 663
   <212> PRT
   <213> Rind
<400> 2
   Met Thr Glu Lys Ala Asn Gly Val Lys Ser Ser Pro Ala Asn Asn His
   1               5                   10                  15
   Asn His His Ala Pro Pro Ala Ile Lys Ala Ser Gly Lys Asp Asp His
               20                  25                  30
   Arg Ala Ser Ser Arg Pro Gln Ser Ala Ala Ala Asp Asp Thr Ser Ser
           35                  40                  45
   Glu Leu Gln Gln Leu Ala Glu Met Asp Ala Pro Gln Gln Arg Arg Gly
       50                  55                  60
   Gly Phe Arg Arg Ile Ala Arg Leu Val Gly Val Leu Arg Glu Trp Ala
   65 70 75 80
   Tyr Arg Asn Phe Arg Glu Glu Glu Pro Arg Pro Asp Ser Phe Leu Glu
   85 90 95
   Arg Phe Arg Gly Pro Glu Leu His Thr Val Thr Thr Gln Gln Gly Asp
   100 105 110
   Gly Lys Gly Asp Lys Asp Gly Glu Gly Lys Gly Thr Lys Lys Lys Phe
   115 120 125
   Glu Leu Phe Val Leu Asp Pro Ala Gly Asp Trp Tyr Tyr Arg Trp Leu
   130 135 140
   Phe Leu Ile Ala Leu Pro Val Leu Tyr Asn Trp Cys Leu Leu Val Ala
   145 150 155 160
   Arg Ala Cys Phe Ser Asp Leu Gln Lys Gly Tyr Tyr Ile Val Trp Leu
   165 170 175
   Val Leu Asp Tyr Val Ser Asp Val Val Tyr Ile Ala Asp Leu Phe Ile
   180 185 190
   Arg Leu Arg Thr Gly Phe Leu Glu Gln Gly Leu Leu Val Lys Asp Thr
   195 200 205
   Lys Lys Leu Arg Asp Asn Tyr Ile His Thr Met Gln Phe Lys Leu Asp
   210 215 220
   Val Ala Ser Ile Ile Pro Thr Asp Leu Ile Tyr Phe Ala Val Gly Ile
   225 230 235 240
   His Asn Pro Glu Val Arg Phe Asn Arg Leu Leu His Phe Ala Arg Met
   245 250 255
   Phe Glu Phe Phe Asp Arg Thr Glu Thr Arg Thr Ser Tyr Pro Asn Ile
   260 265 270
   Phe Arg Ile Ser Asn Leu Ile Leu Tyr Ile Leu Ile Ile Ile His Trp
   275 280 285
   Asn Ala Cys Ile Tyr Tyr Ala Ile Ser Lys Ser Ile Gly Phe Gly Val
   290 295 300
   Asp Thr Trp Val Tyr Pro Asn Ile Thr Asp Pro Glu Tyr Gly Tyr Leu
   305 310 315 320
   Ser Arg Glu Tyr Ile Tyr Cys Leu Tyr Trp Ser Thr Leu Thr Leu Thr
   325 330 335
   Thr Ile Gly Glu Thr Pro Pro Pro Val Lys Asp Glu Glu Tyr Leu Phe
   340 345 350
   Val Ile Phe Asp Phe Leu Ile Gly Val Leu Ile Phe Ala Thr Ile Val
   355 360 365
   Gly Asn Val Gly Ser Met Ile Ser Asn Met Asn Ala Thr Arg Ala Glu
   370 375 380
   Phe Gln Ala Lys Ile Asp Ala Val Lys His Tyr Met Gln Phe Arg Lys
   385 390 395 400
   Val Ser Lys Glu Met Glu Ala Lys Val Ile Arg Trp Phe Asp Tyr Leu
   405 410 415
   Trp Thr Asn Lys Lys Ser Val Asp Glu Arg Glu Val Leu Lys Asn Leu
   420 425 430
   Pro Ala Lys Leu Arg Ala Glu Ile Ala Ile Asn Val His Leu Ser Thr
   435 440 445
   Leu Lys Lys Val Arg Ile Phe Gln Asp Cys Glu Ala Gly Leu Leu Val
   450 455 460
   Glu Leu Val Leu Lys Leu Arg Pro Gln Val Phe Ser Pro Gly Asp Tyr
   465 470 475 480
   Ile Cys Arg Lys Gly Asp Ile Gly Lys Glu Met Tyr Ile Ile Lys Glu
   485 490 495
   Gly Lys Leu Ala Val Val Ala Asp Asp Gly Val Thr Gln Tyr Ala Leu
   500 505 510
   Leu Ser Ala Gly Ser Cys Phe Gly Glu Ile Ser Ile Leu Asn Ile Lys
   515 520 525
   Gly Ser Lys Met Gly Asn Arg Arg Thr Ala Asn Ile Arg Ser Leu Gly
   530 535 540
   Tyr Ser Asp Leu Phe Cys Leu Ser Lys Asp Asp Leu Met Glu Ala Val
   545 550 555 560
   Thr Glu Tyr Pro Asp Ala Lys Arg Val Leu Glu Glu Arg Gly Arg Glu
   565 570 575
   Ile Leu Met Lys Glu Gly Leu Leu Asp Glu Asn Glu Val Ala Ala Ser
   580 585 590
   Met Glu Val Asp Val Gln Glu Lys Leu Glu Gln Leu Glu Thr Asn Met
   595 600 605
   Asp Thr Leu Tyr Thr Arg Phe Ala Arg Leu Leu Ala Glu Tyr Thr Gly
   610 615 620
   Ala Gln Gln Lys Leu Lys Gln Arg Ile Thr Val Leu Glu Thr Lys Met
   625 630 635 640
   Lys Gln Asn Asn Glu Asp Asp Ser Leu Ser Asp Gly Met Asn Ser Pro
   645 650 655
   Glu Pro Pro Ala Glu Lys Pro
   660
<210> 3
   <211> 1989
   <212> DNA
   <213> Rind
<220>
   <221> CDS
   <222> (1)..(1989)
   <223> Nukleinsäuresequenz der genetisch veränderten T537M-α3-Untereinheit des CNG-Kanals aus Rind
<400> 3
   atg aca gaa aaa gcc aat ggc gtg aag agc tcc cca gcc aat aac cac    48
   Met Thr Glu Lys Ala Asn Gly Val Lys Ser Ser Pro Ala Asn Asn His
     1               5                  10                  15
   aac cac cat gcc cct cct gcc atc aag gcc agt ggc aaa gat gac cac    96
   Asn His His Ala Pro Pro Ala Ile Lys Ala Ser Gly Lys Asp Asp His
                20                  25                  30
   agg gcc agc agc cgg cca cag tct gct gct gct gat gac acc tcc tca   144
   Arg Ala Ser Ser Arg Pro Gln Ser Ala Ala Ala Asp Asp Thr Ser Ser
            35                  40                  45
   gag cta cag caa ctg gca gag atg gat gcc ccc cag cag agg agg ggt   192
   Glu Leu Gln Gln Leu Ala Glu Met Asp Ala Pro Gln Gln Arg Arg Gly
        50                  55                  60
   ggc ttc cgc agg att gcc cgc ctg gtg ggg gtc ctc aga gag tgg gct   240
   Gly Phe Arg Arg Ile Ala Arg Leu Val Gly Val Leu Arg Glu Trp Ala
    65                  70                  75                  80
   tac agg aac ttc cgt gag gag gag cct aga cct gac tca ttc ctt gag   288
   Tyr Arg Asn Phe Arg Glu Glu Glu Pro Arg Pro Asp Ser Phe Leu Glu
                    85                  90                  95
   cgt ttc cgg ggg cct gag ctc cac acc gtg aca aca caa caa gga gac   336
   Arg Phe Arg Gly Pro Glu Leu His Thr Val Thr Thr Gln Gln Gly Asp
               100                 105                 110
   ggc aaa ggc gac aag gac ggc gag ggc aag ggc acc aag aag aag ttt   384
   Gly Lys Gly Asp Lys Asp Gly Glu Gly Lys Gly Thr Lys Lys Lys Phe
           115                 120                 125
   gaa ctc ttt gtc ttg gac cca gcc ggg gac tgg tac tac cgc tgg ctt   432
   Glu Leu Phe Val Leu Asp Pro Ala Gly Asp Trp Tyr Tyr Arg Trp Leu
       130                 135                 140
   ttt ctc att gcc ttg ccc gtc ctc tac aac tgg tgc cta ttg gtg gcc   480
   Phe Leu Ile Ala Leu Pro Val Leu Tyr Asn Trp Cys Leu Leu Val Ala
   145                 150                 155                 160
   aga gcc tgc ttc agt gac ctg cag aaa ggc tac tac ata gtg tgg ctg   528
   Arg Ala Cys Phe Ser Asp Leu Gln Lys Gly Tyr Tyr Ile Val Trp Leu
   165 170 175
   gtg ctg gat tac gtc tca gat gtg gtc tac atc gca gac ctc ttc atc 576
   Val Leu Asp Tyr Val Ser Asp Val Val Tyr Ile Ala Asp Leu Phe Ile
   180 185 190
   cga ctg cgc aca ggt ttc ttg gag cag ggg cta ctg gtg aaa gac acc 624
   Arg Leu Arg Thr Gly Phe Leu Glu Gln Gly Leu Leu Val Lys Asp Thr
   195 200 205
   aag aag ttg cgg gac aac tac atc cac acc atg cag ttt aag ctg gat 672
   Lys Lys Leu Arg Asp Asn Tyr Ile His Thr Met Gln Phe Lys Leu Asp
   210 215 220
   gtg gcc tcc atc atc cct aca gac ctg atc tat ttt gct gtg ggg atc 720
   Val Ala Ser Ile Ile Pro Thr Asp Leu Ile Tyr Phe Ala Val Gly Ile
   225 230 235 240
   cat aac cct gag gtg cgc ttc aac cgc ctg cta cac ttt gcc cgc atg 768
   His Asn Pro Glu Val Arg Phe Asn Arg Leu Leu His Phe Ala Arg Met
   245 250 255
   ttt gag ttc ttt gac cgc act gag aca cgc acc agc tac ccc aac atc 816
   Phe Glu Phe Phe Asp Arg Thr Glu Thr Arg Thr Ser Tyr Pro Asn Ile
   260 265 270
   ttc cga ata agc aac ctg atc ctc tac atc ttg atc atc att cac tgg 864
   Phe Arg Ile Ser Asn Leu Ile Leu Tyr Ile Leu Ile Ile Ile His Trp
   275 280 285
   aat gcc tgc atc tac tat gcc atc tcc aag tcc atc ggc ttt ggg gta 912
   Asn Ala Cys Ile Tyr Tyr Ala Ile Ser Lys Ser Ile Gly Phe Gly Val
   290 295 300
   gac acc tgg gtt tac ccc aac atc act gac cct gag tat ggc tac ctg 960
   Asp Thr Trp Val Tyr Pro Asn Ile Thr Asp Pro Glu Tyr Gly Tyr Leu
   305 310 315 320
   tct agg gag tac atc tat tgc ctt tac tgg tct aca ctg acc ctc acc 1008
   Ser Arg Glu Tyr Ile Tyr Cys Leu Tyr Trp Ser Thr Leu Thr Leu Thr
   325 330 335
   acc att ggg gag aca cca ccc cct gta aag gat gag gag tac ctg ttt 1056
   Thr Ile Gly Glu Thr Pro Pro Pro Val Lys Asp Glu Glu Tyr Leu Phe
               340                 345                 350
   gtc atc ttt gac ttc ctg att ggt gtc ctc atc ttt gcc acc atc gtg    1104
   Val Ile Phe Asp Phe Leu Ile Gly Val Leu Ile Phe Ala Thr Ile Val
           355                 360                 365
   gga aat gtg ggc tcc atg atc tcc aac atg aat gcc acc cgg gct gag    1152
   Gly Asn Val Gly Ser Met Ile Ser Asn Met Asn Ala Thr Arg Ala Glu
       370                 375                 380
   ttc cag gcc aag att gat gct gtc aaa cat tat atg cag ttc cga aag    1200
   Phe Gln Ala Lys Ile Asp Ala Val Lys His Tyr Met Gln Phe Arg Lys
   385                 390                 395                 400
   gtc agc aag gag atg gaa gcc aag gtc att agg tgg ttt gac tac ttg    1248
   Val Ser Lys Glu Met Glu Ala Lys Val Ile Arg Trp Phe Asp Tyr Leu
                   405                 410                 415
   tgg acc aat aag aag agt gta gat gag cga gaa gtc ctc aaa aac ctg    1296
   Trp Thr Asn Lys Lys Ser Val Asp Glu Arg Glu Val Leu Lys Asn Leu
               420                 425                 430
   cca gca aag ctc agg gct gag ata gcc atc aac gtc cac ctg tcc aca    1344
   Pro Ala Lys Leu Arg Ala Glu Ile Ala Ile Asn Val His Leu Ser Thr
           435                 440                 445
   ctc aag aaa gtg cgc atc ttt cag gac tgt gag gct ggc ctg ctg gtg    1392
   Leu Lys Lys Val Arg Ile Phe Gln Asp Cys Glu Ala Gly Leu Leu Val
       450                 455                 460
   gaa ctg gta tta aag ctc cgg cct cag gtc ttt agc cct ggg gac tac    1440
   Glu Leu Val Leu Lys Leu Arg Pro Gln Val Phe Ser Pro Gly Asp Tyr
   465                 470                 475                 480
   att tgc cgc aag ggg gat att ggg aag gag atg tac ata atc aag gag    1488
   Ile Cys Arg Lys Gly Asp Ile Gly Lys Glu Met Tyr Ile Ile Lys Glu
                   485                 490                 495
   gga aaa ttg gca gtg gtg gct gat gac ggt gtc act cag tat gcc ctg    1536
   Gly Lys Leu Ala Val Val Ala Asp Asp Gly Val Thr Gln Tyr Ala Leu
               500                 505                 510
   ctc tcg gct ggg agt tgc ttt gga gag atc agt atc ctt aat att aag   1584
   Leu Ser Ala Gly Ser Cys Phe Gly Glu Ile Ser Ile Leu Asn Ile Lys
           515                 520                 525
   ggc agc aaa atg ggc aat cgg cgc atg gcc aac atc cgc agt ctt ggc   1632
   Gly Ser Lys Met Gly Asn Arg Arg Met Ala Asn Ile Arg Ser Leu Gly
       530                 535                 540
   tac tct gat ctg ttc tgc ttg tcc aag gat gat ctt atg gaa gct gtg   1680
   Tyr Ser Asp Leu Phe Cys Leu Ser Lys Asp Asp Leu Met Glu Ala Val
   545                 550                 555                 560
   act gag tac cct gat gcc aag agg gtc ttg gag gag aga ggc cgg gag   1728
   Thr Glu Tyr Pro Asp Ala Lys Arg Val Leu Glu Glu Arg Gly Arg Glu
                   565                 570                 575
   att ctg atg aag gag ggc ttg ttg gat gag aat gag gtg gca gcc agc   1776
   Ile Leu Met Lys Glu Gly Leu Leu Asp Glu Asn Glu Val Ala Ala Ser
               580                 585                 590
   atg gag gta gat gtg cag gaa aag cta gaa cag ctg gag acc aac atg   1824
   Met Glu Val Asp Val Gln Glu Lys Leu Glu Gln Leu Glu Thr Asn Met
           595                 600                 605
   gac acc ttg tac act cgt ttt gcc cgc ctg ctg gcc gag tac acg gga   1872
   Asp Thr Leu Tyr Thr Arg Phe Ala Arg Leu Leu Ala Glu Tyr Thr Gly
       610                 615                 620
   gcc cag cag aag ctc aag cag cgc atc aca gtt ttg gaa acg aag atg   1920
   Ala Gln Gln Lys Leu Lys Gln Arg Ile Thr Val Leu Glu Thr Lys Met
   625                 630                 635                 640
   aag cag aat aat gag gat gac tcc ctg tca gat ggg atg aac agc cca   1968
   Lys Gln Asn Asn Glu Asp Asp Ser Leu Ser Asp Gly Met Asn Ser Pro
                   645                 650                 655
   gag cca cct gcc gag aag cca                                       1989
   Glu Pro Pro Ala Glu Lys Pro
               660
<210> 4
   <211> 663
   <212> PRT
   <213> Rind
<400> 4
   Met Thr Glu Lys Ala Asn Gly Val Lys Ser Ser Pro Ala Asn Asn His
   1 5 10 15
   Asn His His Ala Pro Pro Ala Ile Lys Ala Ser Gly Lys Asp Asp His
   20 25 30
   Arg Ala Ser Ser Arg Pro Gln Ser Ala Ala Ala Asp Asp Thr Ser Ser
   35 40 45
   Glu Leu Gln Gln Leu Ala Glu Met Asp Ala Pro Gln Gln Arg Arg Gly
   50 55 60
   Gly Phe Arg Arg Ile Ala Arg Leu Val Gly Val Leu Arg Glu Trp Ala
   65 70 75 80
   Tyr Arg Asn Phe Arg Glu Glu Glu Pro Arg Pro Asp Ser Phe Leu Glu
   85 90 95
   Arg Phe Arg Gly Pro Glu Leu His Thr Val Thr Thr Gln Gln Gly Asp
   100 105 110
   Gly Lys Gly Asp Lys Asp Gly Glu Gly Lys Gly Thr Lys Lys Lys Phe
   115 120 125
   Glu Leu Phe Val Leu Asp Pro Ala Gly Asp Trp Tyr Tyr Arg Trp Leu
   130 135 140
   Phe Leu Ile Ala Leu Pro Val Leu Tyr Asn Trp Cys Leu Leu Val Ala
   145 150 155 160
   Arg Ala Cys Phe Ser Asp Leu Gln Lys Gly Tyr Tyr Ile Val Trp Leu
   165 170 175
   Val Leu Asp Tyr Val Ser Asp Val Val Tyr Ile Ala Asp Leu Phe Ile
   180 185 190
   Arg Leu Arg Thr Gly Phe Leu Glu Gln Gly Leu Leu Val Lys Asp Thr
   195 200 205
   Lys Lys Leu Arg Asp Asn Tyr Ile His Thr Met Gln Phe Lys Leu Asp
   210 215 220
   Val Ala Ser Ile Ile Pro Thr Asp Leu Ile Tyr Phe Ala Val Gly Ile
   225 230 235 240
   His Asn Pro Glu Val Arg Phe Asn Arg Leu Leu His Phe Ala Arg Met
   245 250 255
   Phe Glu Phe Phe Asp Arg Thr Glu Thr Arg Thr Ser Tyr Pro Asn Ile
   260 265 270
   Phe Arg Ile Ser Asn Leu Ile Leu Tyr Ile Leu Ile Ile Ile His Trp
   275 280 285
   Asn Ala Cys Ile Tyr Tyr Ala Ile Ser Lys Ser Ile Gly Phe Gly Val
   290 295 300
   Asp Thr Trp Val Tyr Pro Asn Ile Thr Asp Pro Glu Tyr Gly Tyr Leu
   305 310 315 320
   Ser Arg Glu Tyr Ile Tyr Cys Leu Tyr Trp Ser Thr Leu Thr Leu Thr
   325 330 335
   Thr Ile Gly Glu Thr Pro Pro Pro Val Lys Asp Glu Glu Tyr Leu Phe
   340 345 350
   Val Ile Phe Asp Phe Leu Ile Gly Val Leu Ile Phe Ala Thr Ile Val
   355 360 365
   Gly Asn Val Gly Ser Met Ile Ser Asn Met Asn Ala Thr Arg Ala Glu
   370 375 380
   Phe Gln Ala Lys Ile Asp Ala Val Lys His Tyr Met Gln Phe Arg Lys
   385 390 395 400
   Val Ser Lys Glu Met Glu Ala Lys Val Ile Arg Trp Phe Asp Tyr Leu
   405 410 415
   Trp Thr Asn Lys Lys Ser Val Asp Glu Arg Glu Val Leu Lys Asn Leu
   420 425 430
   Pro Ala Lys Leu Arg Ala Glu Ile Ala Ile Asn Val His Leu Ser Thr
               435                 440                 445
   Leu Lys Lys Val Arg Ile Phe Gln Asp Cys Glu Ala Gly Leu Leu Val
       450                 455                 460
   Glu Leu Val Leu Lys Leu Arg Pro Gln Val Phe Ser Pro Gly Asp Tyr
   465                 470                 475                 480
   Ile Cys Arg Lys Gly Asp Ile Gly Lys Glu Met Tyr Ile Ile Lys Glu
                   485                 490                 495
   Gly Lys Leu Ala Val Val Ala Asp Asp Gly Val Thr Gln Tyr Ala Leu
               500                 505                 510
   Leu Ser Ala Gly Ser Cys Phe Gly Glu Ile Ser Ile Leu Asn Ile Lys
           515                 520                 525
   Gly Ser Lys Met Gly Asn Arg Arg Met Ala Asn Ile Arg Ser Leu Gly
       530                 535                 540
   Tyr Ser Asp Leu Phe Cys Leu Ser Lys Asp Asp Leu Met Glu Ala Val
   545                 550                 555                 560
   Thr Glu Tyr Pro Asp Ala Lys Arg Val Leu Glu Glu Arg Gly Arg Glu
                   565                 570                 575
   Ile Leu Met Lys Glu Gly Leu Leu Asp Glu Asn Glu Val Ala Ala Ser
               580                 585                 590
   Met Glu Val Asp Val Gln Glu Lys Leu Glu Gln Leu Glu Thr Asn Met
           595                 600                 605
   Asp Thr Leu Tyr Thr Arg Phe Ala Arg Leu Leu Ala Glu Tyr Thr Gly
       610                 615                 620
   Ala Gln Gln Lys Leu Lys Gln Arg Ile Thr Val Leu Glu Thr Lys Met
   625                 630                 635                 640
   Lys Gln Asn Asn Glu Asp Asp Ser Leu Ser Asp Gly Met Asn Ser Pro
                   645                 650                 655
   Glu Pro Pro Ala Glu Lys Pro
               660
<210> 5
   <211> 1989
   <212> DNA
   <213> Rind
<220>
   <221> CDS
   <222> (1)..(1989)
   <223> Nukleinsäuresequenz der genetisch veränderten T537V-α3-Untereinheit des CNG-Kanals aus Rind
<400> 5
   atg aca gaa aaa gcc aat ggc gtg aag agc tcc cca gcc aat aac cac      48
   Met Thr Glu Lys Ala Asn Gly Val Lys Ser Ser Pro Ala Asn Asn His
   1               5                   10                  15
   aac cac cat gcc cct cct gcc atc aag gcc agt ggc aaa gat gac cac      96
   Asn His His Ala Pro Pro Ala Ile Lys Ala Ser Gly Lys Asp Asp His
               20                  25                  30
   agg gcc agc agc cgg cca cag tct gct gct gct gat gac acc tcc tca      144
   Arg Ala Ser Ser Arg Pro Gln Ser Ala Ala Ala Asp Asp Thr Ser Ser
           35                  40                  45
   gag cta cag caa ctg gca gag atg gat gcc ccc cag cag agg agg ggt      192
   Glu Leu Gln Gln Leu Ala Glu Met Asp Ala Pro Gln Gln Arg Arg Gly
       50                  55                  60
   ggc ttc cgc agg att gcc cgc ctg gtg ggg gtc ctc aga gag tgg gct      240
   Gly Phe Arg Arg Ile Ala Arg Leu Val Gly Val Leu Arg Glu Trp Ala
   65                  70                  75                  80
   tac agg aac ttc cgt gag gag gag cct aga cct gac tca ttc ctt gag      288
   Tyr Arg Asn Phe Arg Glu Glu Glu Pro Arg Pro Asp Ser Phe Leu Glu
                   85                  90                  95
   cgt ttc cgg ggg cct gag ctc cac acc gtg aca aca caa caa gga gac      336
   Arg Phe Arg Gly Pro Glu Leu His Thr Val Thr Thr Gln Gln Gly Asp
               100                 105                 110
   ggc aaa ggc gac aag gac ggc gag ggc aag ggc acc aag aag aag ttt   384
   Gly Lys Gly Asp Lys Asp Gly Glu Gly Lys Gly Thr Lys Lys Lys Phe
           115                 120                 125
   gaa ctc ttt gtc ttg gac cca gcc ggg gac tgg tac tac cgc tgg ctt   432
   Glu Leu Phe Val Leu Asp Pro Ala Gly Asp Trp Tyr Tyr Arg Trp Leu
       130                 135                 140
   ttt ctc att gcc ttg ccc gtc ctc tac aac tgg tgc cta ttg gtg gcc   480
   Phe Leu Ile Ala Leu Pro Val Leu Tyr Asn Trp Cys Leu Leu Val Ala
   145                 150                 155                 160
   aga gcc tgc ttc agt gac ctg cag aaa ggc tac tac ata gtg tgg ctg   528
   Arg Ala Cys Phe Ser Asp Leu Gln Lys Gly Tyr Tyr Ile Val Trp Leu
                   165                 170                 175
   gtg ctg gat tac gtc tca gat gtg gtc tac atc gca gac ctc ttc atc   576
   Val Leu Asp Tyr Val Ser Asp Val Val Tyr Ile Ala Asp Leu Phe Ile
               180                 185                 190
   cga ctg cgc aca ggt ttc ttg gag cag ggg cta ctg gtg aaa gac acc   624
   Arg Leu Arg Thr Gly Phe Leu Glu Gln Gly Leu Leu Val Lys Asp Thr
           195                 200                 205
   aag aag ttg cgg gac aac tac atc cac acc atg cag ttt aag ctg gat   672
   Lys Lys Leu Arg Asp Asn Tyr Ile His Thr Met Gln Phe Lys Leu Asp
       210                 215                 220
   gtg gcc tcc atc atc cct aca gac ctg atc tat ttt gct gtg ggg atc   720
   Val Ala Ser Ile Ile Pro Thr Asp Leu Ile Tyr Phe Ala Val Gly Ile
   225                 230                 235                 240
   cat aac cct gag gtg cgc ttc aac cgc ctg cta cac ttt gcc cgc atg   768
   His Asn Pro Glu Val Arg Phe Asn Arg Leu Leu His Phe Ala Arg Met
                   245                 250                 255
   ttt gag ttc ttt gac cgc act gag aca cgc acc agc tac ccc aac atc   816
   Phe Glu Phe Phe Asp Arg Thr Glu Thr Arg Thr Ser Tyr Pro Asn Ile
               260                 265                 270
   ttc cga ata agc aac ctg atc ctc tac atc ttg atc atc att cac tgg   864
   Phe Arg Ile Ser Asn Leu Ile Leu Tyr Ile Leu Ile Ile Ile His Trp
   275 280 285
   aat gcc tgc atc tac tat gcc atc tcc aag tcc atc ggc ttt ggg gta 912
   Asn Ala Cys Ile Tyr Tyr Ala Ile Ser Lys Ser Ile Gly Phe Gly Val
   290 295 300
   gac acc tgg gtt tac ccc aac atc act gac cct gag tat ggc tac ctg 960
   Asp Thr Trp Val Tyr Pro Asn Ile Thr Asp Pro Glu Tyr Gly Tyr Leu
   305 310 315 320
   tct agg gag tac atc tat tgc ctt tac tgg tct aca ctg acc ctc acc 1008
   Ser Arg Glu Tyr Ile Tyr Cys Leu Tyr Trp Ser Thr Leu Thr Leu Thr
   325 330 335
   acc att ggg gag aca cca ccc cct gta aag gat gag gag tac ctg ttt 1056
   Thr Ile Gly Glu Thr Pro Pro Pro Val Lys Asp Glu Glu Tyr Leu Phe
   340 345 350
   gtc atc ttt gac ttc ctg att ggt gtc ctc atc ttt gcc acc atc gtg 1104
   Val Ile Phe Asp Phe Leu Ile Gly Val Leu Ile Phe Ala Thr Ile Val
   355 360 365
   gga aat gtg ggc tcc atg atc tcc aac atg aat gcc acc cgg gct gag 1152
   Gly Asn Val Gly Ser Met Ile Ser Asn Met Asn Ala Thr Arg Ala Glu
   370 375 380
   ttc cag gcc aag att gat gct gtc aaa cat tat atg cag ttc cga aag 1200
   Phe Gln Ala Lys Ile Asp Ala Val Lys His Tyr Met Gln Phe Arg Lys
   385 390 395 400
   gtc agc aag gag atg gaa gcc aag gtc att agg tgg ttt gac tac ttg 1248
   Val Ser Lys Glu Met Glu Ala Lys Val Ile Arg Trp Phe Asp Tyr Leu
   405 410 415
   tgg acc aat aag aag agt gta gat gag cga gaa gtc ctc aaa aac ctg 1296
   Trp Thr Asn Lys Lys Ser Val Asp Glu Arg Glu Val Leu Lys Asn Leu
   420 425 430
   cca gca aag ctc agg gct gag ata gcc atc aac gtc cac ctg tcc aca 1344
   Pro Ala Lys Leu Arg Ala Glu Ile Ala Ile Asn Val His Leu Ser Thr
   435 440 445
   ctc aag aaa gtg cgc atc ttt cag gac tgt gag gct ggc ctg ctg gtg 1392
   Leu Lys Lys Val Arg Ile Phe Gln Asp Cys Glu Ala Gly Leu Leu Val
   450 455 460
   gaa ctg gta tta aag ctc cgg cct cag gtc ttt agc cct ggg gac tac 1440
   Glu Leu Val Leu Lys Leu Arg Pro Gln Val Phe Ser Pro Gly Asp Tyr
   465 470 475 480
   att tgc cgc aag ggg gat att ggg aag gag atg tac ata atc aag gag 1488
   Ile Cys Arg Lys Gly Asp Ile Gly Lys Glu Met Tyr Ile Ile Lys Glu
   485 490 495
   gga aaa ttg gca gtg gtg gct gat gac ggt gtc act cag tat gcc ctg 1536
   Gly Lys Leu Ala Val Val Ala Asp Asp Gly Val Thr Gln Tyr Ala Leu
   500 505 510
   ctc tcg gct ggg agt tgc ttt gga gag atc agt atc ctt aat att aag 1584
   Leu Ser Ala Gly Ser Cys Phe Gly Glu Ile Ser Ile Leu Asn Ile Lys
   515 520 525
   ggc agc aaa atg ggc aat cgg cgc gtc gcc aac atc cgc agt ctt ggc 1632
   Gly Ser Lys Met Gly Asn Arg Arg Val Ala Asn Ile Arg Ser Leu Gly
   530 535 540
   tac tct gat ctg ttc tgc ttg tcc aag gat gat ctt atg gaa gct gtg 1680
   Tyr Ser Asp Leu Phe Cys Leu Ser Lys Asp Asp Leu Met Glu Ala Val
   545 550 555 560
   act gag tac cct gat gcc aag agg gtc ttg gag gag aga ggc cgg gag 1728
   Thr Glu Tyr Pro Asp Ala Lys Arg Val Leu Glu Glu Arg Gly Arg Glu
   565 570 575
   att ctg atg aag gag ggc ttg ttg gat gag aat gag gtg gca gcc agc 1776
   Ile Leu Met Lys Glu Gly Leu Leu Asp Glu Asn Glu Val Ala Ala Ser
   580 585 590
   atg gag gta gat gtg cag gaa aag cta gaa cag ctg gag acc aac atg 1824
   Met Glu Val Asp Val Gln Glu Lys Leu Glu Gln Leu Glu Thr Asn Met
   595 600 605
   gac acc ttg tac act cgt ttt gcc cgc ctg ctg gcc gag tac acg gga 1872
   Asp Thr Leu Tyr Thr Arg Phe Ala Arg Leu Leu Ala Glu Tyr Thr Gly
   610 615 620
   gcc cag cag aag ctc aag cag cgc atc aca gtt ttg gaa acg aag atg 1920
   Ala Gln Gln Lys Leu Lys Gln Arg Ile Thr Val Leu Glu Thr Lys Met
   625 630 635 640
   aag cag aat aat gag gat gac tcc ctg tca gat ggg atg aac agc cca 1968
   Lys Gln Asn Asn Glu Asp Asp Ser Leu Ser Asp Gly Met Asn Ser Pro
   645 650 655
   gag cca cct gcc gag aag cca 1989
   Glu Pro Pro Ala Glu Lys Pro
   660
<210> 6
   <211> 663
   <212> PRT
   <213> Rind
<400> 6
   Met Thr Glu Lys Ala Asn Gly Val Lys Ser Ser Pro Ala Asn Asn His
   1 5 10 15
   Asn His His Ala Pro Pro Ala Ile Lys Ala Ser Gly Lys Asp Asp His
   20 25 30
   Arg Ala Ser Ser Arg Pro Gln Ser Ala Ala Ala Asp Asp Thr Ser Ser
   35 40 45
   Glu Leu Gln Gln Leu Ala Glu Met Asp Ala Pro Gln Gln Arg Arg Gly
   50 55 60
   Gly Phe Arg Arg Ile Ala Arg Leu Val Gly Val Leu Arg Glu Trp Ala
   65 70 75 80
   Tyr Arg Asn Phe Arg Glu Glu Glu Pro Arg Pro Asp Ser Phe Leu Glu
   85 90 95
   Arg Phe Arg Gly Pro Glu Leu His Thr Val Thr Thr Gln Gln Gly Asp
   100 105 110
   Gly Lys Gly Asp Lys Asp Gly Glu Gly Lys Gly Thr Lys Lys Lys Phe
   115 120 125
   Glu Leu Phe Val Leu Asp Pro Ala Gly Asp Trp Tyr Tyr Arg Trp Leu
   130 135 140
   Phe Leu Ile Ala Leu Pro Val Leu Tyr Asn Trp Cys Leu Leu Val Ala
   145 150 155 160
   Arg Ala Cys Phe Ser Asp Leu Gln Lys Gly Tyr Tyr Ile Val Trp Leu
   165 170 175
   Val Leu Asp Tyr Val Ser Asp Val Val Tyr Ile Ala Asp Leu Phe Ile
   180 185 190
   Arg Leu Arg Thr Gly Phe Leu Glu Gln Gly Leu Leu Val Lys Asp Thr
   195 200 205
   Lys Lys Leu Arg Asp Asn Tyr Ile His Thr Met Gln Phe Lys Leu Asp
   210 215 220
   Val Ala Ser Ile Ile Pro Thr Asp Leu Ile Tyr Phe Ala Val Gly Ile
   225 230 235 240
   His Asn Pro Glu Val Arg Phe Asn Arg Leu Leu His Phe Ala Arg Met
   245 250 255
   Phe Glu Phe Phe Asp Arg Thr Glu Thr Arg Thr Ser Tyr Pro Asn Ile
   260 265 270
   Phe Arg Ile Ser Asn Leu Ile Leu Tyr Ile Leu Ile Ile Ile His Trp
   275 280 285
   Asn Ala Cys Ile Tyr Tyr Ala Ile Ser Lys Ser Ile Gly Phe Gly Val
   290 295 300
   Asp Thr Trp Val Tyr Pro Asn Ile Thr Asp Pro Glu Tyr Gly Tyr Leu
   305 310 315 320
   Ser Arg Glu Tyr Ile Tyr Cys Leu Tyr Trp Ser Thr Leu Thr Leu Thr
   325 330 335
   Thr Ile Gly Glu Thr Pro Pro Pro Val Lys Asp Glu Glu Tyr Leu Phe
   340 345 350
   Val Ile Phe Asp Phe Leu Ile Gly Val Leu Ile Phe Ala Thr Ile Val
   355 360 365
   Gly Asn Val Gly Ser Met Ile Ser Asn Met Asn Ala Thr Arg Ala Glu
   370 375 380
   Phe Gln Ala Lys Ile Asp Ala Val Lys His Tyr Met Gln Phe Arg Lys
   385 390 395 400
   Val Ser Lys Glu Met Glu Ala Lys Val Ile Arg Trp Phe Asp Tyr Leu
   405 410 415
   Trp Thr Asn Lys Lys Ser Val Asp Glu Arg Glu Val Leu Lys Asn Leu
   420 425 430
   Pro Ala Lys Leu Arg Ala Glu Ile Ala Ile Asn Val His Leu Ser Thr
   435 440 445
   Leu Lys Lys Val Arg Ile Phe Gln Asp Cys Glu Ala Gly Leu Leu Val
   450 455 460
   Glu Leu Val Leu Lys Leu Arg Pro Gln Val Phe Ser Pro Gly Asp Tyr
   465 470 475 480
   Ile Cys Arg Lys Gly Asp Ile Gly Lys Glu Met Tyr Ile Ile Lys Glu
   485 490 495
   Gly Lys Leu Ala Val Val Ala Asp Asp Gly Val Thr Gln Tyr Ala Leu
   500 505 510
   Leu Ser Ala Gly Ser Cys Phe Gly Glu Ile Ser Ile Leu Asn Ile Lys
   515 520 525
   Gly Ser Lys Met Gly Asn Arg Arg Val Ala Asn Ile Arg Ser Leu Gly
   530 535 540
   Tyr Ser Asp Leu Phe Cys Leu Ser Lys Asp Asp Leu Met Glu Ala Val
   545 550 555 560
   Thr Glu Tyr Pro Asp Ala Lys Arg Val Leu Glu Glu Arg Gly Arg Glu
   565 570 575
   Ile Leu Met Lys Glu Gly Leu Leu Asp Glu Asn Glu Val Ala Ala Ser
   580 585 590
   Met Glu Val Asp Val Gln Glu Lys Leu Glu Gln Leu Glu Thr Asn Met
   595 600 605
   Asp Thr Leu Tyr Thr Arg Phe Ala Arg Leu Leu Ala Glu Tyr Thr Gly
   610 615 620
   Ala Gln Gln Lys Leu Lys Gln Arg Ile Thr Val Leu Glu Thr Lys Met
   625 630 635 640
   Lys Gln Asn Asn Glu Asp Asp Ser Leu Ser Asp Gly Met Asn Ser Pro
   645 650 655
   Glu Pro Pro Ala Glu Lys Pro
   660
<210> 7
   <211> 26
   <212> DNA
   <213> Künstliche Sequenz
<220>
   <223> Beschreibung der künstlichen Sequenz: primer
<220>
   <223> Mutagenese-Primer zur Herstellung der T537M-α3-Untereinheit
<400> 7
   cgacgcatgg cgaacatccg cagtct 26
<210> 8
   <211> 26
   <212> DNA
   <213> Künstliche Sequenz
<220>
   <223> Beschreibung der künstlichen Sequenz: primer
<220>
   <223> Mutagenese-Primer zur Herstellung der T537V-α3-Untereinheit
<400> 8
   cgacgcgtcg cgaacatccg cagtct 26
<210> 9
   <211> 30
   <212> DNA
   <213> Künstliche Sequenz
<220>
   <223> Beschreibung der künstlichen Sequenz: primer
<220>
   <223> PCR-Primer #1817
<400> 9
   ttggctgcag ctattatggc ttctcggcag 30
<210> 10
   <211> 27
   <212> DNA
   <213> Künstliche Sequenz
<220>
   <223> Beschreibung der künstlichen Sequenz: primer
<220>
   <223> flankierender Primer #1813
<400> 10
   gtcggatcct ccacactcaa gaaagtg 27
<210> 11
   <211> 30
   <212> DNA
   <213> Künstliche Sequenz
<220>
   <223> Beschreibung der künstlichen Sequenz: primer
<220>
   <223> Primer #843 für die Klonierung des CRF-Rezeptors
<400> 11
   agcgggatcc accatgggac ggcgcccgca 30
<210> 12
   <211> 18
   <212> DNA
   <213> Künstliche Sequenz
<220>
   <223> Beschreibung der künstlichen Sequenz: primer
<220>
   <223> Primer #842 für die Klonierung des CRF-Rezeptors
<400> 12
   ggcctggagc tcacactg 18

## Beschreibung

Die vorliegende Erfindung betrifft genetisch modifizierte Nukleinsäuren, vorzugsweise DNAs, die für zyklisch-Nukleotid-gesteuerte lonenkanäle (CNG-Kanäle) kodieren und die entsprechenden Proteine und deren Verwendung.

Chemische Substanzen wie beispielsweise Hormone oder Neurotransmitter können als sogenannte "primäre Botenstoffe" (Liganden) an die Membran von Zellen binden und dadurch vielfältige biochemisch-physiologische Reaktionen im Zellinneren auslösen, mit denen die Zellen auf ihre Umgebung reagieren können. Dieser Prozess wird durch eine Vielzahl membranständiger Rezeptoren vermittelt, an die Liganden spezifisch und direkt binden können. Er steht am Anfang von verschiedenen, zum Teil äußerst komplexen Signalübertragungskaskaden. Über solche Kaskaden steuern und regulieren Rezeptoren die Aktivität verschiedener zellulärer Proteine (Effektorproteine). Diese Effektorproteine ihrerseits können wiederum die Konzentration von intrazellulären Botenstoffen, den sogenannten "sekundären Botenstoffen" regulieren. Erst durch solche sekundären Botenstoffe wird eine Vielzahl von physiologischen Reaktionen gesteuert, wie beispielsweise die Synthese und die Ausschüttung von Hormonen und Neurotransmittern, die Zellteilung und das Zellwachstum, sowie die Erregung und Erregbarkeit von neuronalen Zellen. Zu den besonders wichtigen sekundären Botenstoffen zählen das zyklische Adenosinmonophosphat (cAMP), das zyklische Guanosinmonophosphat (cGMP) und Cm²⁺-lo-nen.
Die intrazelluläre Konzentration der sekundären Botenstoffe wird zumeist durch Signalübertragungskaskaden reguliert, in der G-Protein-gekoppelte Rezeptoren in der Membran von Zellen (GPCRs) ein extrazelluläres Signal registrieren, dann entsprechende G-Proteine aktivieren und diese wiederum die Aktivität der entsprechenden Effektorproteine entweder stimulieren oder inhibieren (Morris AJ and Malbon CC. (1999) Physiological regulation of G protein-linked signaling. Physiol Rev., 79, 1373-1430). Darüber hinaus kann die Aktivität jeder einzelnen Komponente im Rahmen von vielfältigen Rückkopplungsmechanismen durch weitere Proteine moduliert werden.
Abweichungen in der physiologisch normalen Konzentration von Liganden oder Störungen im Ablauf einer Signalübertragungskaskade, die beispielsweise durch die Fehlfunktion einer daran beteiligten Komponente ausgelöst wird, können schwere Erkrankungen auslösen. Weil GPCRs eine besonders wichtige Schnittstelle zwischen extrazellulärem und intrazellulärem Medium der Zelle darstellen, indem sie als Bindestelle bzw. Angriffsort einer großen Vielzahl von körpereigenen (endogenen) und körperfremden (exogenen) chemischen Substanzen dienen und zudem in großer Vielfalt in nahezu jedem lebenswichtigen Organ oder Gewebe wichtige physiologische Prozesse steuern, sind sie von überragendem Interesse für medizinisch-pharmakologische Interventionen. Besonders wichtige Indikationsgebiete dabei sind Erkrankungen des zentralen und peripheren Nervensystems, des Herz-Kreislaufsystems und der inneren Organe.
Therapeutisches Ziel der Pharmaindustrie ist es, pharmakologische Wirkstoffe zu entwickeln, die die Zielproteine aktivieren (Agonisten), inhibieren (Antagonisten) oder aber in ihrer Aktivität modulieren. Dies erfordert zusätzlich die eingehende funktionelle Charakterisierung der entsprechenden Zielproteine. Dafür sind in den letzten Jahren verschiedene Methoden entwickelt worden, die sich zum Teil deutlich in ihrer Flexibilität, aber auch in der Aussagekraft der damit erzielten Ergebnisse, sowie ihrer Robustheit und ihrer Effizienz hinsichtlich Geschwindigkeit, Arbeitsaufwand und Kosten unterscheiden.
Nachdem inzwischen die komplementäre DNA einer Vielzahl von Rezeptoren kloniert worden ist, und die Rezeptoren funktionell in Zellsystemen exprimiert werden können, werden die Untersuchungen hauptsächlich an heterolog exprimierten Rezeptoren durchgeführt. Der Stand der Technik hinsichtlich der verwendeten Strategien und Methoden, sowie deren Anwendung sind beispielsweise in Artikeln von Hertzberg RP and und Pope AJ. (2000) High-throughput screening: new technology for the 21st century. Curr Opin Chem Biol., 4, 445-451, Howard AD, Mac Allister G, Feighner SD, Liu Q, Nargund RP, van der Ploeg LH, and Patchett AA. (2001) Orphan G-protein-coupled receptors and natural ligand discovery. Trends Pharmacol Sci., 22, 132-140., Civelli O, Nothacker HP, Saito Y, Wang Z, Lin SH, and Reinscheid RK. (2001) Novel neurotransmitters as natural ligands of orphan G-protein-coupled receptors. Trends Neurosci., 24, 230-237., sowie den darin angegebenen Referenzen beschrieben.
Über 60 % der bekannten GPCRs regulieren die intrazelluläre Konzentration des sekundären Botenstoffs cAMP. Für die Untersuchungen der Wirkung von chemischen Substanzen auf solche GPCRs und die entsprechenden Effektorproteine existieren verschiedene Methoden.
Einige dieser Verfahren basieren auf direkten, zumeist radiochemischen Messungen der intrazellulären cAMP-Konzentration. Dazu werden beispielsweise die Zellen stimuliert, nach einer bestimmten Zeit biochemisch aufgebrochen und die Änderung der cAMP-Konzentration bestimmt. Diese Meßverfahren sind zwar sehr empfindlich, aber zumeist inhärent langsam, kostenintensiv und zeitaufwendig. Zudem kann die Änderung der intrazellulären cAMP-Konzentration nicht in Echtzeit verfolgt werden. Wichtige charakteristische Eigenschaften, wie Geschwindigkeit und Verlauf einer Aktivierung oder Inhibierung können nur mit einem erheblichen zusätzlichen Aufwand durch eine Vielzahl von zusätzlichen Messungen ermittelt werden. Ein Vorteil dieser Methoden ist hingegen, daß nicht nur die Effekte von Wirkstoffen auf GPCRs untersucht werden können, sondern auch auf die Effektorproteine, die die intrazelluläre cAMP-Konzentration regulieren.
Ein weiteres Verfahren, mit dem man intrazelluläre Änderungen der cAMP- bzw. der cGMP-Konzentration messen kann, macht sich die Eigenschaften von membranständigen CNG-Kanälen zu nutze. Zyklisch-Nukleotid-gesteuerte lonenkanäle (CNG-Kanäle) sind membranständige Proteine, die nachfolgend beschriebene Merkmale und Eigenschaften besitzen (Finn JT., Grunwald ME, and Yau KW. (1996) Cyclic nucleotide-gated ion channels: an extended family with diverse functions. Annu Rev Physio., 58, 395-426; Richards MJ and Gordon SE. (2000) Cooperativity and cooperation in cyclic nucleotide-gated ion channels. Biochemistry, 39, 14003-14011). CNG-Kanäle bestehen (1) aus vermutlich 4 oder 5 Untereinheiten (α- und/oder β-Untereinheiten), die (2) jeweils sechsmal die Membran durchspannen und (3) jeweils eine Bindestelle für zyklische Nukleotide am Carboxy-terminalen intrazellulären Ende besitzen. CNG-Kanäle werden (4) direkt und dosisabhängig durch cAMP oder cGMP aktiviert, bilden (5) eine wässrige Pore in der Membran mit einer nur wenig selektiven Leitfähigkeit für einwertige Kationen und sind (6) ebenfalls permeabel für zweiwertige Kationen wie beispielsweise Ca²⁺-lonen.
Als "Bindestelle für zyklische Nukleotide" wird der Abschnitt in den CNG-Kanal Untereinheiten bezeichnet, an den die zyklischen Nukleotide cAMP und cGMP dosisabhängig binden können. Die Aminosäuresequenz in diesem Abschnitt bestimmt maßgeblich die Empfindlichkeit eines CNG-Kanals für cAMP bzw. cGMP (Sensitivität).
Als "Leitfähigkeit" wird die Eigenschaft von Ionenkanälen bezeichnet, mehr oder weniger selektiv den Einstrom von Ionen vom Zelläußeren in das Zellinnere oder den Ausstrom von Ionen aus dem Zellinneren nach außen zu ermöglichen. Dazu bilden die lonenkanäle eine Öffnung in der Membran (wässrige Pore), durch die, abhängig vom Aktivierungszustand der Kanäle, Ionen entsprechend dem Konzentrationsgefälle ein- oder ausströmen können.
In heterologen Expressionssystemen ist es möglich, funktionelle CNG-Kanäle aus gleichen α-Untereinheiten (Homooligomere; Kaupp UB, Niidome T, Tanabe T, Terada S, Bönigk W, Stühmer W. Cook NJ, Kanagawa K, Matsuo H, Hirose T, Miyata T, and Numa S. (1989) Primary structure and functional expression from complementary DNA of the rod photoreceptor cyclic GMP-gated channel. Nature, 342, 762-766), aus unterschiedlichen α-Untereinheiten (Heterooligomere; Bradley J, Li J, Davidson N, Lester HA, and Zinn K. (1994) Heteromeric olfactory cyclic nucleotide-gated channels: a subunit that confers increased sensitivity to cAMP. Proc. Natl Acad Sci USA, 91, 8890-8894.; Liman ER and Buck LB. (1994) A second subunit of the olfactory cyclic nucleotide-gated channel confers high sensitivity to cAMP. Neuron 13, 611-621), sowie als Heterooligomere aus α- und β-Untereinheiten zu exprimieren (Chen TY, Peng YW, Dhallan RS, Ahamed B, Reed RR, and Yau KW. (1993) A new subunit of the cyclic nucleotide-gated cation channel in retinal rods. Nature, 362, 764-767). Die β-Untereinheiten alleine können keine funktionellen Kanäle bilden, sondern besitzen ausschließlich modulatorische Funktionen in heterooligomeren CNG-Kanälen (Chen TY, Peng YW, Dhallan RS, Ahamed B, Reed RR, and Yau KW. (1993) A new subunit of the cyclic nucleotide-gated cation channel in retinal rods. Nature, 362, 764-767). Wenn cAMP oder cGMP an CNG-Kanäle bindet, öffnen die Kanäle dosisabhängig und Ionen strömen in die Zelle. Die Aktivierung der CNG-Kanäle führt unter physiologischen Bedingungen zu einer erhöhten Ca²⁺-Leitfähigkeit der Kanäle und bewirkt so das Ansteigen der intrazellulären Ca²⁺-Konzentration. Eine solche Konzentrationsänderung kann mit optischen Ca²⁺-Meßverfahren gemessen werden. Diese lonenkanäle könnten also prinzipiell als zellulärer cAMP-Sensor für die Untersuchung und Charakterisierung aller Rezeptoren und intrazellulären Proteine eingesetzt werden, die die intrazelluläre cAMP-Konzentration regulieren. Im Vergleich zu den direkten cAMP-Messungen ist dieses Verfahren sehr schnell, effektiv und kostengünstig. Es erlaubt einen hohen Durchsatz an Tests pro Tag und ermöglicht Messungen in Echtzeit. Prinzipiell eignet sich dieses Verfahren deshalb besonders gut für die pharmakologische Wirkstoffsuche.
In den Schriften US 6,001,581 und WO 98/58074, sowie von Gotzes F. (1995) Dissertation. ISSN 0944-2952 wird die Verwendung von CNG-Kanälen als cAMP-Sensor beschrieben, die aus der α3-Untereinheit aus dem Riechepithel der Nase bestehen. Solche CNG-Kanäle besitzen jedoch mehrere entscheidende Nachteile, wenn sie bei der pharmazeutischen Wirkstoffsuche als zelluläre cAMP-Sensoren eingesetzt werden. Diese CNG-Kanäle werden bereits durch 2 µM cGMP, aber erst durch 80 µM cAMP halbmaximal aktiviert (Dhallan RS, Yau KW, Schrader KA, and Reed RR. (1990) Primary structure and functional expression of a cyclic nucleotide-activated channel from olfactory neurons. Nature, 347, 184-187; Ludwig J, Margalit T, Eismann E, Lancet D, and Kaupp UB. (1990) Primary structure of cAMP-gated channel from bovine olfactory epithelium. FEBS Lett., 270, 24-29). Da die Änderungen der intrazellulären cAMP-Konzentration aber normalerweise nur wenige µM betragen, eignen sich solche CNG-Kanäle grundsätzlich zwar als cGMP-Sensoren, aber nur schlecht als cAMP-Sensoren. Zudem können die Messungen der cAMP-Konzentration schon durch geringfügige Schwankungen der intrazellulären cGMP-Konzentration gestört werden.
Heterooligomere CNG-Kanäle aus α3-, α4- und β1b-Untereinheiten hingegen werden schon bei einer cAMP-Konzentration von etwa 4 µM halbmaximal aktiviert (K_{1/2}-Wert), während sich der K_{1/2}-Wert für cGMP im Vergleich zu den homooligomeren Kanälen nur unwesentlich ändert (Bönigk W, Bradley J, Müller F, Sesti F, Boekhoff I, Ronnett GV, Kaupp UB, and Frings S., (1999) The native rat olfactory cyclic nucleotide-gated channel is composed of three distinct subunits. J Neurosci., 19, 5332-5347. Prinzipiell eignen sich solche CNG-Kanäle ausgezeichnet als zelluläre cAMP-Sensoren. Die Expression solcher Kanäle in heterologen Zellsystemen ist jedoch nachteilhaft sehr arbeits- und zeitaufwendig. Zudem können geringfüge Schwankungen der intrazellulären cGMP-Konzentration die Funktion als cAMP-Sensor stören.
CNG-Kanäle, die nur aus α-Untereinheiten bestehen, aber dennoch eine hohe Sensitivität für cAMP-besitzen, kann man aber auch mit molekularbiologischen Methoden herstellen: 1991 wurde eine genetisch modifizierte α3-Untereinheit des CNG-Kanals aus Rind beschrieben, bei der das Threonin an Position 537 gegen ein Serin (T537S) ausgetauscht wurde (Altenhofen W, Ludwig J, Eismann E, Kraus W, Bönigk W, and Kaupp UB. (1991) Control of ligand specificity in cyclic nucleotide-gated channel from rod photoreceptors and olfactory epithelium. Proc Natl Acad Sci USA, 88, 9868-9872). Diese Untereinheit bildet CNG-Kanäle, die mit 14 µM cAMP halbmaximal aktiviert werden. Das Threonin T537 liegt in dem Sequenzabschnitt der α3-Untereinheit, der maßgeblich an der Bindung der zyklischen Nukleotide beteiligt ist. Offensichtlich ist die Aminosäure an dieser Position besonders wichtig für die Sensitivität der CNG-Kanäle (Altenhofen W, Ludwig J, Eismann E, Kraus W, Bönigk W, and Kaupp UB. (1991) Control of ligand specificity in cyclic nucleotide-gated channel from rod photoreceptors and olfactory epithelium. Proc Natl Acad Sci USA, 88, 9868-9872.) Mit dieser Mutation T537S steigt allerdings auch die Empfindlichkeit der Kanäle für cGMP an (K_{1/2} = 0,7 µM). Solche Kanäle (T537S-Mutanten) sind zwar mit geringem Aufwand heterolog zu exprimieren, als cAMP-Sensor sind sie allerdings noch störanfälliger für geringfügige Schwankungen der intrazellulären cGMP-Konzentration als die heterooligomeren Kanäle. Zudem ist die Empfindlichkeit für cAMP noch nicht groß genug, um auch geringe Schwankungen der intrazellulären cAMP-Konzentration zuverlässig zu registrieren.
Ferner sind Mutationen in der α3-Untereinheit des CNG-Kanals bekannt, die die Sensitivität für cAMP erhöhen und zusätzlich die Empfindlichkeit für cGMP vermindern. (Rich TC, Tse TE, Rohan DG, Schaack J, and Karpen JW. (2001) In vivo assessment of local phosphodiesterase activity using tailored cyclic nucleotide-gated channels as cAMP sensors. J Gen Physol., 118; 63-78). CNG-Kanäle aus der Ratten α3-Untereinheit, in der das Cystein an Position 460 (C460) gegen ein Tryptophan (W) und zusätzlich das Glutamat an Position 583 (E583) gegen ein Methionin (M) ausgetauscht sind (C460W/E583M-Mutante), werden durch etwa 1,2 µM cAMP, aber erst durch 12 µM cGMP halbmaximal aktiviert. Als weitere, für die Ligandenselektivität maßgebliche Aminosäure wurde ein Aspartat (D604 in der CNGA1-Untereinheit) in einer alpha-helikalen Region, der sogenannten C-Helix der Bindestelle für zyklische Nukleotide identifiziert (Varnum et al., Neuron. 1995 Sep;15(3):619-25). Es wurde vermutet, dass der negativ geladene Aminosäurerest an dieser Position elektrostatisch bedingt verhindert, dass der CNG-Kanal sensitiv auf cAMP reagiert: Durch den Austausch des Aspartats gegen eine neutrale Aminosäure (D604Q, D604N oder D604T) oder eine nichtpolare Aminosäure (D604M oder D604V) wurde die Sensitivität des untersuchten CNG-Kanals für cAMP enorm gesteigert bzw. sogar die Ligandenselektivität zugunsten des cAMP umgekehrt (Varnum et al., Neuron. 1995 Sep;15(3):619-25).

Aufgabe der Erfindung war es, CNG-Kanäle als cAMP-Sensoren zu entwickeln, die ein ähnliche Empfindlichkeit für cAMP bzw. cGMP aufweisen wie die C460W/E583M-Mutante, aber nur an einer Position genetisch modifiziert sind. Solche CNG-Kanäle können in einfachen und schnellen zellulären Meßsystemen effizient und universell für die pharmazeutische Wirkstoffsuche, aber auch für die Charakterisierung von pharmakologischen oder potentiell pharmakologischen Zielproteinen verwendet werden.

Diese Aufgabe wird durch CNG-Kanäle aus α3-Untereinheiten gelöst, die an der Position, die dem Threonin T537 in der α3-Untereinheit aus Rind entspricht, so verändert sind, daß sie eine höhere Sensitivität für cAMP und/oder eine höhere Selektivität für cAMP gegenüber cGMP im Vergleich zum Wildtyp gemäß SEQ ID NO 2 aufweisen, wobie die Aminosäure die dem Threonin T537 in der alpha 3-Untereinheit des Rindes gemäß SEQ ID NO: 2 entspricht, durch Methionin ersetzt ist.

Diese lonenkanäle besitzen eine ähnliche Empfindlichkeit für cAMP bzw. cGMP wie die C460W/E583M-Mutante.

Gegenstand der Erfindung ist außerdem ein Verfahren zur Herstellung dieser CNG-Kanäle aus Rind und/oder anderen Organismen, dadurch gekennzeichnet, dass die genetisch modifizierten zyklisch-Nukleotid-gesteuerte lonenkanäle (CNG-Kanäle) aus alpha3-Untereinheiten, die an der Position, die dem Threonin T537 in der alpha3-Untereinheit aus Rind gemäß SEQ ID NO:2 entspricht, so verändert sind, daß sie eine höhere Sensitivität für cAMP und/oder eine höhere Selektivität für cAMP gegenüber cGMP im Vergleich zum Wildtyp gemäß SEQ ID NO:2 aufweisen dadurch gekennzeichnet, daß die Aminosäure, die dem Threonin T537 in der alpha3-Untereinheit des Rindes gemäß SEQ ID NO:2 entspricht, durch Methionin ersetzt ist

Gegenstand der Erfindung sind auch Expressionsvektoren, die die Nukleinsäuren für die modifizierten CNG-Kanäle gemäß den Ansprüchen 1 bis 4 enthalten. Gegenstand der Erfindung sind ebenfalls Zelllinien, die mit den beschriebenen Expressionsvektoren transformiert werden und die CNG-Kanäle exprimieren können. Besonders bevorzugt sind Zelllinien, die entweder GPCRs, Adenylatzyklasen, Phosphodiesterasen oder andere Proteine, die intrazelluläre cAMP-Konzentration regulieren, gemeinsam mit einem modifizierten CNG-Kanal heterolog koexprimieren können.

Gegenstand der Erfindung ist ferner ein Verfahren zur Herstellung der genannten Zelllinien, bei dem eine Transformation mittels Expressionsvektoren durchgeführt wird. Erfindungsgemäß werden vorzugsweise die Gene für die Proteine in den Expressionsvektor kloniert und anschließend die Transformation der Zelllinien durchgeführt.

Erfindungsgemäß werden bevorzugt CNG-Kanäle aus α3-Untereinheiten verwendet. Es kommen aber auch andere Untereinheiten aus Rind oder anderen Organismen in Betracht.

Bei den erfindungsgemäß verwendeten Untereinheiten ist vorzugsweise die Aminosäure, die dem Threonin an der Position T537 in der α3-Untereinheit des Rindes entspricht, durch eine andere, von Serin verschiedene Aminosäure ersetzt. Besonders bevorzugt sind hierbei solche Untereinheiten, bei denen das Threonin durch Methionin oder Valin ersetzt ist. In den SEQ ID NO 3 und 4 bzw. SEQ ID NO 5 und 6 sind derartig geänderte Untereinheiten am Beispiel der α3-Untereinheit aus Rind dargestellt.

Die erfindungsgemäßen lonenkanäle eignen sich vor allem als zelluläre cAMP-Sensoren für die Messung der intrazellulären cAMP-Konzentration. Ebenso sind sie geeignet für die Bestimmung der Wirkung von Liganden, Agonisten und Antagonisten auf G-Protein-gekoppelte Rezeptoren (GPCRs), die die intrazelluläre cAMP-Konzentration regulieren. Außerdem können sie zur Bestimmung der Wirkung von Aktivatoren und Inhibitoren auf Adenylatzyklasen und Phosphodiesterasen (Effektorproteine), die die intrazelluläre cAMP-Konzentration regulieren, eingesetzt werden.

zudem beschrieben ist die Verwendung von zellulären Meßsystemen mit Nukleinsäuren und den entsprechenden Proteinen für die Bestimmung der Wirkung von chemischen Substanzen, die die Aktivität von zellulären Komponenten beeinflussen, die die intrazelluläre cAMP-Konzentration direkt oder indirekt regulieren.

Die zellulären Meßsysteme können universell und flexibel als cAMP-Sensoren für die pharmazeutische Wirkstoffsuche und Wirkstoffcharakterisierung, sowie für die Charakterisierung von pharmakologisch relevanten Proteinen eingesetzt werden. Diese umfassen alle G-Protein-gekoppelten Rezeptoren, Adenylatzyklasen, Phosphodiesterasen sowie alle anderen Proteine, die an cAMP-Signalwegen beteiligt sind.

Als zelluläre cAMP-Sensoren können anstelle der T537M-Mutante oder der C460W/E583M-Mutante prinzipiell aber auch andere genetisch modifizierte CNG-Kanäle aus alpha3-Untereinheiten aus Rind und/oder anderen Organismen hergestellt und verwendet werden, die
(i) eine ähnlich hohe oder höhere Sensitivität für cAMP,
(ii) eine ähnlich hohe oder höhere Sensitivität für cAMP, oder aber
(iii) eine ähnlich hohe oder höhere Sensitivität und zusätzlich eine ähnlich hohe
oder höhere Selektivität für cAMP besitzen.

Diese genetisch modifizierten CNG-Kanäle können eine homooligomere Zusammensetzung aus gleichen oder eine heterooligomere Zusammensetzung aus verschiedenen Untereinheiten aufweisen.

Solche CNG-Kanäle können (1) α3-Untereinheiten anderer Organismen, (2) andere CNG-Kanal-Untereinheiten aus Rind oder anderen Organismen enthalten, sowie (3) eine homooligomere oder heterooligomere Zusammensetzung aus diesen Untereinheiten aufweisen. Diese Untereinheiten können (4) jeweils an der Position genetisch modifiziert sein, die der Position T537 in der α3-Untereinheit des CNG-Kanals aus Rind entspricht. An dieser Position kann das Threonin durch ein Methionin oder ein Valin, oder aber durch eine andere Aminosäure (ausgenommen Serin) ersetzt sein. Solche Untereinheiten können (5) weitere genetische Modifikationen an anderen Positionen aufweisen. Ferner können diese CNG-Kanäle (6) chimäre Untereinheiten enthalten, die an dieser Position in gleicher Weise genetisch modifiziert sind.

Darunter fallen auch genetisch modifizierte alpha3-Untereinheiten von CNG-Kanälen aus Rind und/oder anderen Organismen, dadurch gekennzeichnet, daß sie eine homooligomere Zusammensetzung aus gleichen alpha3-Untereinheiten oder eine heterooligomere Zusammensetzung aus verschiedenen alpha3-Untereinheiten aus Rind und/oder anderen Organismen aufweisen.

"An der Position genetisch modifiziert, die der Position T537 in der α3-Untereinheit des CNG-Kanals aus Rind entspricht" bedeutet folgendes: Die verschiedenen Untereinheiten von CNG-Kanälen (z.B. α1, α2, α3 und α4), bzw. gleiche Untereinheiten von CNG-Kanälen aus verschiedenen Organismen, wie beispielsweise die α3-Untereinheit aus Rind und aus Ratte, weisen eine hohe Sequenzähnlichkeit zueinander auf. Dennoch unterscheidet sich zumeist die Lage der strukturell und funktionell wichtigen Abschnitte in der Aminosäuresequenz der Untereinheiten geringfügig. Ein Fachmann kann jedoch durch einen Vergleich der Sequenzen die einander entsprechenden Sequenzenabschnitte bzw. Aminosäuren identifizieren. Das Threonin T537 in der Bindestelle für zyklische Nukleotide in der α3-Untereinheit aus Rind entspricht beispielsweise dem Threonin T539 in der α3-Untereinheit aus Ratte oder dem Threonin T560 in der α1-Untereinheit aus Rind.

"Weitere genetische Modifikationen" bedeutet, daß zusätzlich zu einer erfindungsgemäßen Modifikationen an mindestens einer anderen Position eine Aminosäure gegen eine andere ausgetauscht oder mindestens einer anderen Position eine Aminosäure deletiert oder hinzugefügt ist.

Als "chimäre Untereinheiten" werden solche CNG-Kanal-Untereinheiten bezeichnet, die aus mindestens zwei unterschiedlichen Teilen von Untereinheiten zusammengesetzt sind, also beispielsweise eine Untereinheit, die aus dem Amino-terminalen Teil der α1-Untereinheit und dem Carboxy-terminalen Teil aus der α3-Untereinheit zusammengesetzt ist. Solche Chimären sind leicht von einem Fachmann mit molekularbiologischen Methoden herzustellen und werden oft benutzt, um bestimmte Eigenschaften eines Proteins mit den Eigenschaften eines anderen Proteins zu kombinieren, oder um bestimmte Eigenschaften auf ein anderes Protein zu übertragen, oder aber um bestimmte Eigenschaften im Vergleich zu den Wildtyp-Proteinen zu verändern. Chimären aus verschiedenen CNG-Kanal-Untereinheiten sind bereits beschrieben worden (Seifert R, Eismann E, Ludwig J, Baumann A, and Kaupp, UB. (1999) Molecular determinants of a Ca2+-binding site in the pore of cyclic nucleotide-gated channels: S5/S6 segments control affinity of intrapore glutamates. EMBO J., 18, 119-130).

In Wirbeltieren sind sechs Gene für Untereinheiten von CNG-Kanälen bekannt (α1-α4, β1, β2). Zusätzlich existieren verschiedene Isoformen dieser Untereinheiten (Sautter A, Zonh X, Hofmann F, and Biel M. (1998) An isoform of the rod photoreceptor cyclic nucleotide-gated channel beta subunit expressed in olfactory neurons. Proc Natl Acad Sci USA, 95, 4696-4701; Bönigk W, Bradley J, Müller F, Sesti F, Boekhoff I, Ronnett GV, Kaupp UB, and Frings S. (1999) The native rat olfactory cycloc nucleotide-gated channel is composed of three distinct subunits. J Neurosci., 19, 5332-5347). Erstmals entdeckt wurde die α1-Untereinheit (Kaupp UB, Niidome T, Tanabe T, Terada S, Bönigk W, Stühmer W, Cook NJ, Kangawa K, Matsuo H, Hirose T, Miyata T, and Numa S. (1989) Primary structure and functional expression from complementary DNA of the rod photoreceptor cyclic GMP-gated channel. Nature, 342, 762-766.) und die β1-Untereinheit (Chen TY, Peng YW, Dhallam RS, Ahamed B, Reed RR, and Yau KW (1993) A new subunit of the cyclic nucleotide-gated cation channel in retinal rods. Nature, 362, 764-767; Körschen HG, Illing M, Seifert R, Sesti F, Williams A, Gotzes S, Colville C, Müller F, Dose A, Godde M, Molday L, Kaupp UB, and Molday RS. (1995) A 240 kDa protein represents the complete beta subunit of the cyclic nucleotide-gated cation channel from rod photoreceptor. Neuron, 15, 627-636) in den Sehstäbchen der Netzhaut, die α2-Untereinheit (Bönigk W, Altenhofen W, Müller F, Dose A, Illing M, Molday RS, and Kaupp UB. (1993) Rod and cone photoreceptor cells express distinct genes for cGMP-gated channels. Neuron, 10, 865-877) und die β2-Untereinheit (Gerstner A, Zong X, Hofmann F, and Biel M. (2000) Molecular cloning and functional characterization of a new modulatory cyclic nucleotide-gated channel subunit from mouse retina. J Neurosci., 20, 1324-1332) in Sehzapfen der Netzhaut, sowie die α3-Untereinheit (Dhallan RS, Yau KW, Schrader KA, and Reed RR. (1990) Primary structure and functional expression of a cyclic nucleotide-activated channel from olfactory neurons. Nature, 347, 184-187; Ludwig J, Margalit T, Eismann E, Lancet D, and Kaupp UB. (1990) Primary structure of cAMP-gated channel from bovine olfactory epithelium. FEBS Lett., 270, 24-29) und die α4-Untereinheit (Bradley J, Li J, Davidson N, Lester HA, and Zinn K.(1994) Heteromeric olfactory cyclic nucleotide-gated channels: a subunit that confers increased sensitivity to cAMP. Proc natl Acad Sci USA, 91, 8890-8894; Liman ER and Buck LB. (1994) A second subunit of the olfactory cyclic nucleotide-gated channel confers high sensitivity to cAMP. Neuron, 13,611-621) in Riechzellen der Nase.

CNG-Kanäle aus diesen Untereinheiten wurden darüber hinaus in zahlreichen anderen neuronalen und nicht-neuronalen Zellen und Geweben nachgewiesen (Richards MJ and Gordon SE. (2000) Cooperativity and cooperation in cyclic nucleotide-gated ion channels. Biochemistry, 39, 14003-14011). Zudem wurden CNG-Kanäle nicht nur in Wirbeltieren gefunden, sondern ebenfalls in Wirbellosen wie beispielsweise in Drosophila melanogaster (Baumann A, Frings S, Godde M, Seifert R, and Kaupp UB. (1994) Primary structure and functional expression of a Drosophila cyclic nucteotide-gated channel present in eyes and antennae. EMBO J., 13, 5040-5050) und Pflanzen (Leng Q, Mercier RW, Yao W, and Berkowitz GA. (1999) Cloning and first functional characterization of a plant cyclic nucleotide-gated cation channel. Plant Physiol., 121, 753-761). Prinzipiell können diese und alle anderen Untereinheiten von CNG-Kanälen erfindungsgemäß modifiziert werden.

Die genetisch modifizierten CNG-Kanäle können in zellulären Testsystemen als cAMP-Sensor für pharmakologische Untersuchungen eingesetzt werden,
(i) um die Wirkung von Liganden, Agonisten und Antagonisten auf membranständige G-Protein gekoppelte Rezeptoren (GPCRs) zu untersuchen, die die intrazelluläre cAMP-Konzentration regulieren,
(ii) um die Wirkung von Aktivatoren und Inhibitoren auf Effektorproteine (Enzyme) zu untersuchen, die cAMP synthetisieren oder hydrolysieren,
(iii) um die Wirkung von Aktivatoren und Inhibitoren auf andere Proteine zu untersuchen, die ebenfalls regulierend in die cAMP-Signalübertragungskaskade eingreifen, aber auch,
(iv) um die Eigenschaften von GPCRs, Effektorproteinen oder anderen Proteinen, die an cAMP-Signalübertragungskaskaden beteiligt sind,
zu untersuchen.

Die erfindungsgemäß als "membranständige G-Protein gekoppelte Rezeptoren" (GPCRs) bezeichneten Proteine gehören zu der phylogenetisch vielfältigsten, äußerst umfangreichen Familie der membranständigen Rezeptoren (Übersichtsartikel: Morris AJ and Malbon CC. (1999) Physiological regulation of G protein-linked signaling. Physio Rev., 79, 1373-1430): Die Familie der GPCRs umfaßt vermutlich deutlich mehr als 1.000 unterschiedliche Mitglieder, die anhand ihrer Sequenzähnlichkeit (Probst WC, Snyder LA, Schuster DI, Brosius J, and Sealfon SC. (1992) Sequence alignment of the G-protein coupled receptor superfamily. DNA Cell Biol., 11, 1-20) oder basierend auf der chemischen Natur ihrer natürlichen Liganden klassifiziert werden können. Eine Zusammenstellung und eine Klassifizierung der bislang bekannten GPCRs findet man beispielsweise in der "GPCRDB"-Datenbank (Hom F, Weare J, Beukers MW, Horsch S, Bairoch A, Chen W, Edvardsen O, Campagne F, and Vriend G. (1998) GPCRDB: an information system for G protein-coupled receptors. Nucleic Acids Res., 26, 275-279). Nachfolgend sind übersichtsweise einige der Vertreter der einzelner Klassen aufgeführt. Zur Klasse A ("Rhodopsin like") gehört das Rhodopsin selbst und verschiedene sequenzverwandte Rezeptoren, die anhand ihrer natürlichen Liganden klassifiziert sind: Dazu gehören Rezeptoren für (1) biogene Amine wie beispielsweise die muscarinischen Acetylcholin-Rezeptoren, adrenerge Rezeptoren, Dopamin-, Histamin-, Serotonin-, Octopamin-Rezeptoren, für (2) Peptide, wie beispielsweise die Angiotensin-, Chemokin-, Endothelin-, Neuropeptid-Rezeptoren, für (3) Hormon-Proteine, wie beispielsweise FSH-Rezeptoren, für (4) Geruchsstoffe, für (5) Prostanoide, wie beispielsweise Prostaglandin-Rezeptoren, oder für (6) Nukleotide, wie beispielsweise Adenosin-Rezeptoren. Zur Klasse B ("Secretin like") gehören die Sekretin-Rezeptoren selber, sowie beispielsweise Rezeptoren für Calcitonin, Glukagon, Diuretische Hormone oder CRF (Corticotropin releasing factor). Zur Klasse C ("Metabotropic glutamate / Pheromone") gehören die metabotropen Rezeptoren selber, sowie GABA-B-Rezeptoren und andere. Weitere Klassen enthalten Rezeptoren aus Pflanzen, Pilzen, Insekten, Bakterien. Sämtlich Klassen enthalten Rezeptoren, deren Funktion bislang noch nicht bekannt ist, bzw. von denen man den natürlichen Liganden noch nicht kennt (orphan Rezeptoren). Von etwa 200 verschiedenen GPCR-Typen kennt man inzwischen jeweils die natürlichen Liganden, weitere etwa 100 GPCR-Typen sind orphan GPCRs. 700 oder mehr GPCRs werden vermutlich durch Geruchsstoffe oder Geschmackstoffe aktiviert. Prinzipiell können alle GPCRs, die die intrazelluläre cAMP-Konzentration regulieren, in heterologen Expressionssystemen mit den erfindungsgemäßen genetisch modifizierten CNG-Kanälen als cAMP-Sensor koexprimiert und pharmakologisch die Wirkung von Liganden, Agonisten und Antagonisten untersucht werden.

Es können auch GCRPs untersucht werden, die normalerweise nicht über stimulatorische oder inhibitorische G-Proteine die intrazelluläre cAMP-Konzentration regulieren. Solche GPCRs können durch genetische Modifikation so verändert werden, daß sie an den cAMP-Signalweg koppeln. Die genetische Modifikation kann beispielsweise durch die Herstellung von chimären-GCRPs erfolgen (Liu J, Conklin BR, Blin N, Yun J, and Wess J. (1995) Identification of a receptor/G-protein contact site critical for signaling specificity and G-protein activation. Proc Natl Acad Sci USA, 92, 11642-11646).

Als "Agonisten" und "Liganden" werden Substanzen bezeichnet, die die GCRP aktivieren.

Als "Antagonisten" hingegen werden Substanzen bezeichnet, die zwar an GCRPs binden, sie aber nicht aktivieren können. Die Wirkung von Liganden oder Agonisten wird durch die Antagonisten dosisabhängig inhibiert.

Zu den "Effektorproteinen", die die intrazelluläre cAMP-Konzentration direkt regulieren, zählen Adenylatzyklasen und Phosphodiesterasen.

"Adenylatzyklasen", deren Aktivität GPCR-vermittelt gesteuert wird, sind große, membranständige Enzyme, die die Bildung von cAMP aus Mg²⁺-Adenosintriphosphat katalysieren und in den meisten Zellen, Geweben und Organen des menschlichen Körpers vorhanden sind (Tang WJ and Hurley JH. (1998) Catalytic mechanism and regulation of mammalian adenylyl cyclases. Mol Pharmacol., 54, 231-240). Insgesamt sind 9 unterschiedliche Klassen dieser Adenylatzyklasen bekannt. Solche Adenylatzyklasen werden auch in den zellulären Testsystemen endogen exprimiert und können durch heterolog exprimierte GPCRs aktiviert werden. Sie spielen deshalb eine entscheidenden Rolle für das Funktionieren des Testsystems. Eine weitere Klasse umfaßt lösliche Adenylatzyklasen, deren Aktivität vermutlich nicht GPCR-vermittelt reguliert wird (Buck J, Sinclair ML, Schapal L, Cann MJ, and Levin LR. (2000) Cytosolic adenylyl cyclase defines a unique signaling molecule in mammals. Proc Natl Acad Sci USA, 96, 79-84). Prinzipiell können alle Adenylatzyklasen in heterologen Expressionssystemen mit den erfindungsgemäßen genetisch modifizierten CNG-Kanälen als cAMP-Sensor koexprimiert und die Wirkung von Inhibitoren und Aktivatoren pharmakologisch untersucht werden.

"Phosphodiesterasen" (PDEs) sind Enzyme im Zellinneren, die cAMP und cGMP zu Adenosinmonophosphat (AMP) bzw. Guanosinmonophosphat (GMP) hydrolysieren (Francis SH, Turko IV, and Corbin JD. (2000) Cyclic nucleotide phosphodiesterases: relating structure and function. Prog Nucleic Acid Res Mol Biol., 65, 1-52). Insgesamt sind 11 unterschiedliche Typen von PDEs bekannt. Manche der PDEs hydrolysieren spezifisch cGMP, andere wiederum cAMP und wiederum andere hydrolysieren sowohl cAMP als auch cGMP. Ebenso wie GPCRs und Adenylatzyklasen werden die PDEs in den meisten Zellen, Geweben und Organen des menschlichen Körpers exprimiert. Im Gegensatz zu den Adenylatzyklasen wird allerdings nur die PDE6, die spezifisch für Photorezeptoren ist, GP-CR-vermittelt aktiviert. Die Aktivität der anderen PDEs wird stattdessen durch verschiedene andere Mechanismen reguliert.
Prinzipiell können alle PDEs, die cAMP hydrolysieren, in heterologen Expressionssystemen mit den erfindungsgemäßen genetisch modifizierten CNG-Kanälen als cAMP-Sensor koexprimiert und die Wirkung von Inhibitoren und Aktivatoren pharmakologisch untersucht werden.

Als "Aktivatoren" bezeichnet man Substanzen, die direkt mit Adenylatzyklasen oder PDEs wechselwirken und dadurch deren enzymatische Aktivität steigern.

Als "Inhibitoren" hingegen bezeichnet man Substanzen, die ebenfalls direkt mit Adenylatzyklasen oder PDEs wechselwirken, aber deren Aktivität vermindern.

Prinzipiell können alle Proteine, die die intrazelluläre cAMP-Konzentration regulieren, in heterologen Expressionssystemen mit den erfindungsgemäßen genetisch modifizierten CNG-Kanälen als cAMP-Sensor koexprimiert und pharmakologisch untersucht werden.

Die zu untersuchenden Proteine können entweder transient oder vorzugsweise stabil in heterologen Expressionssystemen exprimiert werden.
"Transiente Expression" bedeutet, daß das heterolog exprimierte Protein nur für einen bestimmten Zeitraum von den Zellen des Expressionssystems exprimiert wird.
"Stabile Expression" bedeutet, daß das eingebrachte Gen stabil in das Genom der Zellen des heterologen Expressionssystems integriert wird. Die so entstandene neue Zelllinie exprimiert das entsprechende Protein in jeder nachfolgenden Zellgeneration.
Für die Expression wird die cDNA, die für das zu untersuchende Proteine kodiert, in einen Expressionsvektor kloniert und in die Zellen eines geeigneten Expressionssystems transformiert.
Als "Expressionsvektoren" bezeichnet man alle Vektoren, mit denen man cDNAs in die entsprechenden Zelllinien einbringen ("transformieren") kann und die entsprechenden Proteine dort funktionell exprimieren kann ("heterologe Expression"). Die Transformation kann vorzugsweise mit dem pcDNA-Vektoren (Invitrogen) durchgeführt werden.
Als "heterologe Expressionssysteme" eignen sich prinzipiell alle eukaryontischen Zellen, wie beispielsweise Hefe-, Aspergillus-, Insekten-, Wirbeltier- und insbesondere Säugetierzellen. Geeignete Zelllinien sind beispielsweise CHO-Zellen (Chinese hamster ovary), zum Beispiel die K1-Linie (ATCC CCL 61), einschließlich der Pro 5 Variante (ATCC CRL 1781), COS-Zellen (African green monkey), zum Beispiel der CV-1 Linie (ATCC CCL 70), einschließlich der COS-1-Variante (ATCC CRL 1650) und der COS-7-Variante (ATCC 1651), BHK-Zellen (Baby hamster kidney) zum Beispiel die Linie BHK-21 (ATCC CCL 10), MRC-5 (ATCC CCL 171), murine L-Zellen, murine NIH/3T3-Zellen (ATCC CRL 1658), murine C127-Zellen (ATCC CRL-1616), menschliche Karzinoma Zellen wie beispielsweise die HeLa-Linie (ATCC CCL 2), Neuroblastom-Zellen der Linien IM-R-32 (ATCC CCL 127), neuro-2A-Zellen (ATCC CLL 131), SK-N-MC-Zellen (ATCC HTB 10) und SK-N-SH-Zellen (ATCC HTB 11), PC12-Zellen (ATCC CRL 1721), sowie Sf9-Zellen (Spodoptera frugiperda) (ATCC CRL 1711). Vorzugsweise werden HEK 293-Zellen (human embryonic kidney) (ATCC CRL 1573), einschließlich der SF-Variante (ATCC 1573.1) verwendet. Besonders bevorzugt ist die Zelllinie DSM ACC 2516.

Die Wirkung von Testsubstanzen auf das zu untersuchende Protein kann mit einem Fluoreszenz-optischen Meßverfahren durchgeführt werden.
Das zu untersuchende Protein wird in einem zellulären Testsystem gemeinsam mit einem cAMP-Sensor heterolog exprimiert und mit Liganden, Agonisten, Antagonisten, Aktivatoren oder Inhibitoren aktiviert bzw. inhibiert. Der cAMP-Sensor registriert die Änderungen der cAMP-Konzentration und Ca²⁺-lonen strömen in stärkerem oder vermindertem Ausmaß in die Zelle ein.

"Fluoreszenz-optisches Meßverfahren" bedeutet, daß die Veränderung der intrazellulären Ca²⁺-Konzentration mit einem fluoreszierenden Ca²⁺-Indikator sichtbar gemacht wird. Mittlerweile sind ein Vielzahl unterschiedlicher Ca²⁺-Indikatoren bekannt (Haugland RP, (1996) Handbook of fluorescent probes and research chemicals. Molecular Probes Inc.). Dazu zählen beispielsweise Fluo-3, Calcium Green, Fura-2 und das erfindungsgemäß bevorzugte Fluo-4 (Molecular Probes). Da solche Indikatoren normalerweise wasserlöslich sind und deshalb nicht die hydrophobe Lipidmembran von Zellen passieren können, werden die Indikatoren statt dessen in Form einer Acetoxymethylester-Verbindung appliziert (Tsien RY. (1981) A non-disruptive technique for loading calcium buffers and indicators into cell. Nature, 290, 527-528). Diese Verbindungen hingegen sind hydrophob und werden von den Zellen aufgenommen. Im Zellinnem wird die Esterbindung von endogenen, intrazellulären Esterasen gespalten und der Indikator liegt wieder in seiner wasserlöslichen Form vor. In dieser Form verbleibt er im Zellinnern, reichert sich dort an und kann so als intrazellulärer Ca²⁺-Indikator eingesetzt werden. Wenn dieser Indikator dann mit Licht einer geeigneten Wellenlänge angeregt wird, zeigt er Fluoreszenz, die von der intrazellulären Ca²⁺-Konzentration abhängig ist. Das Ausmaß (Amplitude) der Fluoreszenz und der zeitliche Verlauf (Kinetik) korrelieren mit dem Ausmaß und dem Verlauf der Aktivierung des untersuchten Proteins und können mit einem sehr guten Signal/Rauschverhältnis mit Fluoreszenzdetektoren in Echtzeit verfolgt und mit einer geeigneten Software grafisch dargestellt werden.

Für die Messung der intrazellulären Ca²⁺-Konzentration können ebenfalls der Aequorin-Proteinkomplex, der aus Apoaequorin und dem chromophoren Co-Faktor Coelenterazin besteht, oder vergleichbare Komplexe als Ca²⁺-Indikatoren eingesetzt werden (Brini M, Pinton P, Pozzan T and Rizzuto R. (1999) Targeted recombinant aequorins: tools for monitoring (Ca2+) in the various compartments of a living cell. Micrsc Res Tech., 46, 380-389). Dazu muß das Apoaequorin gemeinsam mit dem erfindungsgemäßen cAMP-Sensor und dem zu untersuchenden Protein in dem zellulären Testsystem heterolog exprimiert werden. Vor den Messungen müssen die Zellen mit Coelenterazin inkubiert werden, sodaß sich Apoaequorin und Coelenterazin zum aktiven Aequorin-Komplex zusammenlagern können. Wenn die intrazelluläre Ca²⁺-Konzentration ansteigt, wird Coelenterazin zu Coelenteramid oxidiert. Bei diesem Vorgang wird CO₂ gebildet und Lumineszenz ausgestrahlt. Nachteilig ist, daß dieser Vorgang irreversibel ist. Die Lumineszenz kann mit einem geeigneten optischen Detektionsgerät registriert werden (Lumineszenz-optisches Meßverfahren). Dieses optische Meßverfahren ist zwar ähnlich empfindlich wie das Fluoreszenz-optische Meßverfahren, für Messungen jedoch, bei denen der Verlauf der Reaktion in Echtzeit verfolgt wird, eignet es sich weniger gut.

Fluoreszenz- oder Lumineszenz-optische Messungen mit einem Testsystem können in Küvetten-Meßgeräten, in Ca²⁺-Imaging-Mikroskopen oder in Fluoreszenz- bzw. Lumineszenzreadem durchgeführt werden.

Erfindungsgemäß bevorzugt werden die Messungen in Vertiefungen von Plastikbehältern (Multiwell-Platten) in Fluoreszenzreadem durchgeführt. Die Zellen können in Suspension oder aber vorzugsweise auf dem Boden der Vertiefungen anhaftend vorgelegt werden. Es können Multiwell-Platten mit einer unterschiedlichen Zahl an Vertiefungen benutzt werden, wie beispielsweise Multiwell-Platten mit 96, 384, 1536 oder mehr Vertiefungen. Die Multiwell-Platten ermöglichen es, in einer einzigen Platte eine Vielzahl von gleichen oder unterschiedlichen Messungen durchzuführen.

"Fluoreszenzreader" bzw. "Lumineszenzreader" sind sehr empfindliche optische Meßgeräte, mit denen die Fluoreszenz bzw. Lumineszenz in Multiwell-Platten gemessen werden kann. In solchen Geräten kann die Wirkung von Liganden, Agonisten, Antagonisten, Aktivatoren oder Inhibitoren sehr schnell und mit einem hohen Durchsatz untersucht werden.

Erfindungsgemäß können die Eigenschaften von GPCRs, Effektorproteinen oder anderen Proteinen, die an cAMP-Signalübertragungskaskaden beteiligt sind, quantitativ mit Fluoreszenz- oder Lumineszenz-optischen Messungen untersucht werden.

Erfindungsgemäß können prinzipiell aber auch Messungen mit einem hohen Durchsatz an Tests pro Tag durchgeführt werden. Es ist somit die Suche nach neuen pharmakologischen Wirkstoffen möglich. Hierbei können bis zu 100.000 Substanzen pro Tag (Hochdurchsatz-Screening, HTS-Screening) bzw. mehr als 100.000 Substanzen pro Tag (Ultra-HTS-Screening, UHTS-Screening) getestet werden.

Mit einem FLIPR384-Fluoreszenzreader (Molecular Devices) beispielsweise können bis zu 384 voneinander unabhängige Messungen gleichzeitig durchgeführt und die Fluoreszenz in Echtzeit verfolgt werden.

Im Folgenden wird die Erfindung anhand der Ausführungsbeispiele und der beiliegenden Abbildungen näher beschrieben:

### Beispiele 1 - 4

Es wurden genetisch modifizierte, homooligomere α3-CNG-Kanäle aus Rind hergestellt, die statt des Threonin T537 des Wildtyp-Kanals ein Methionin (T537M) bzw. ein Valin (T537V) in ihrer Sequenz aufweisen. Dazu wurde die Nukleinsäure, die für die α3-Untereinheit des CNG-Kanals aus Rind kodiert (SEQ ID NO 1), mit molekularbiologischen Methoden durch zielgerichtete Mutagenese an der Position 537 verändert ("genetisch modifiziert"), so daß eine Nukleinsäure entstand, die für die T537M-Mutante kodiert (SEQ ID NO 3), und eine andere, die für die T537V-Mutante (SEQ ID NO 5) kodiert (s. Methoden).
Die SEQ ID NO 2, 4 und 6 zeigen die Aminosäuresequenzen des Wildtyp-Kanals, der T537M-Mutante bzw. der T537V-Mutante.

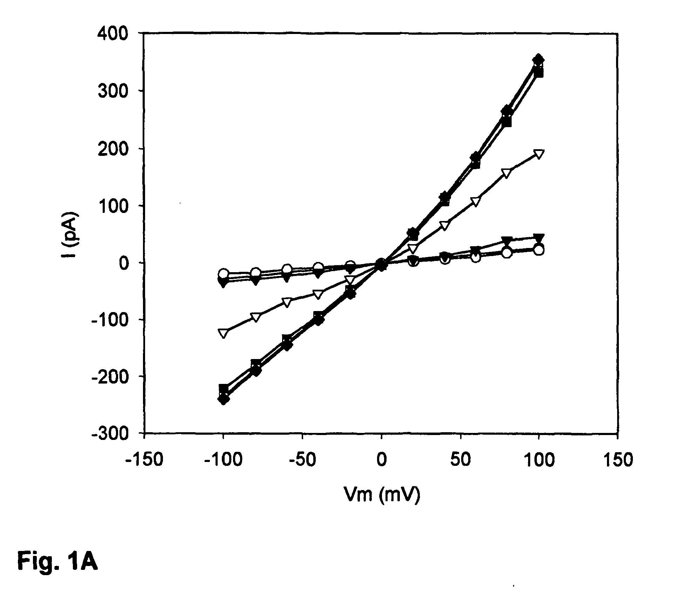

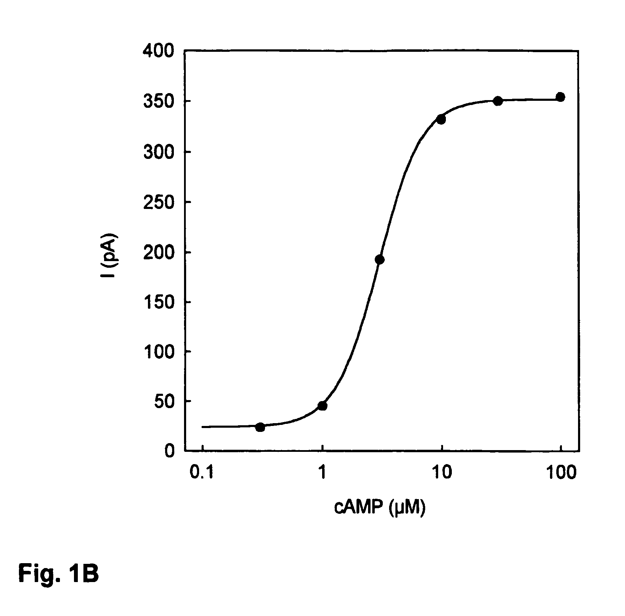

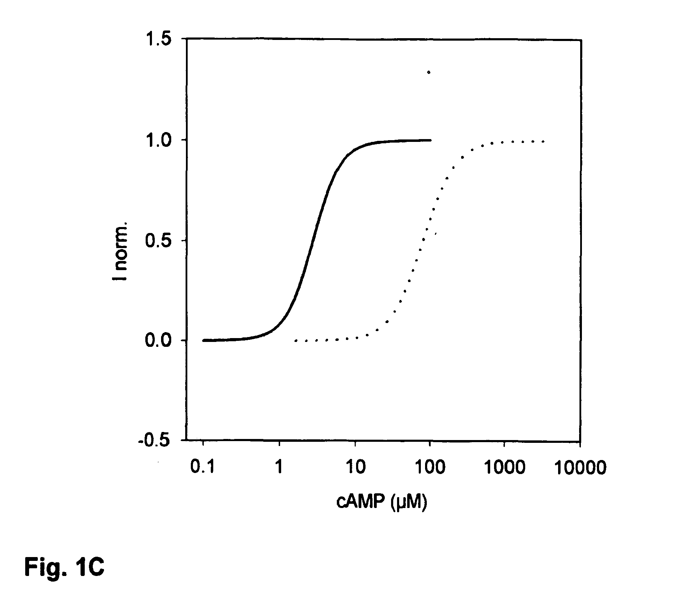

Die Sensitivität und Selektivität der T537M-Mutante und der T537V-Mutante für cAMP und cGMP wurde mit elektrophysiologischen Methoden bestimmt (s. Methoden) und mit den Eigenschaften des Wildtyp-Kanals verglichen. Dafür wurden die entsprechenden Nukleinsäuren in den Expressionsvektor pcDNA3.1 (Invitrogen) kloniert (s. Methoden). Mit den Expressions-Konstrukten wurden dann die Zellen der menschlichen embryonalen Nierenzelllinie 293 (HEK293-Zellen) transformiert und die entsprechenden Proteine darin funktionell entweder transient oder stabil exprimiert (s. Methoden). Die Ergebnisse der Untersuchungen sind in den Abbildungen 1 - 4 dargestellt.
Die Abbildungen 1A, 2A, 3A und 4A zeigen jeweils die Strom-Spannungs-(IV-) Beziehung der heterolog exprimierten genetisch modifizierten CNG-Kanäle in Gegenwart von verschiedenen Konzentrationen an cAMP (1A, 3A) bzw. cGMP (2A, 4A). Sie zeigen jeweils eine für CNG-Kanäle typische Strom-Spannung-Beziehung in Gegenwart von cAMP bzw. cGMP und belegen, daß die Kanäle funktionell in den HEK293-Zellen exprimiert werden.
Die Abhängigkeit der mittleren Ströme von der cAMP- bzw. cGMP-Konzentration ist in den Abbildungen 1-4 B jeweils in Form einer Dosis-Wirkungs-Beziehung dargestellt. Die Ergebnisse dieser Messungen wurden benutzt, um die Sensitivität der CNG-Kanäle für cAMP bzw. für cGMP zu berechnen. Die Konzentration an cAMP bzw. cGMP bei Vₘ= +100 mV, bei der die Kanäle die Hälfte der maximal möglichen Stromes leiten (K_{1/2}-Wert), wird als Maß für die Sensitivität der CNG-Kanäle benutzt. Je niedriger der K_{1/2}-Wert ist, desto höher ist die Empfindlichkeit des Kanals für das entsprechende zyklische Nukleotid.
Die Abbildungen 1 B (T537M-Mutante) und 3B (T537V-Mutante) zeigen jeweils die Dosis-Wirkungsbeziehung für cAMP; die Abbildung 2B (T537M-Mutante) und 4B (T537V-Mutante) hingegen zeigen jeweils die Dosis-Wirkungsbeziehung für cGMP.
In den Abbildungen 1C (T537M-Mutante) und 3C (T537V-Mutante) wird jeweils die normierte Dosis-Wirkungsbeziehung der Mutanten mit der des α3-Wildtyp-Kanal für cAMP verglichen; in den Abbildungen 2C (T537M-Mutante) und 4C (T537V-Mutante) hingegen wird jeweils die normierte Dosis-Wirkungsbeziehung mit der des α3-Wildtyp-Kanal für cGMP verglichen.
Nachstehende Tabelle faßt die Sensitivitäts-Eigenschaften der genetisch modifizierten CNG-Kanäle und des Wildtyp-Kanals zusammen. Aufgelistet sind die K_{1/2}-Werte für cAMP und für cGMP.

**Tabelle 1**

| | | | K_{1/2} für cAMP | K_{1/2} für cGMP |
|---|---|---|---|---|
| W | 1. | Wildtyp | 80 µM | 1,6 µM |
| | 2. | T537S | 14 µM | 0,7 µM |
| | 3. | **T537M** | **2,7 µM** | **14,9 µM** |
| | 4. | **T537V** | **34 µM** | **241 µM** |

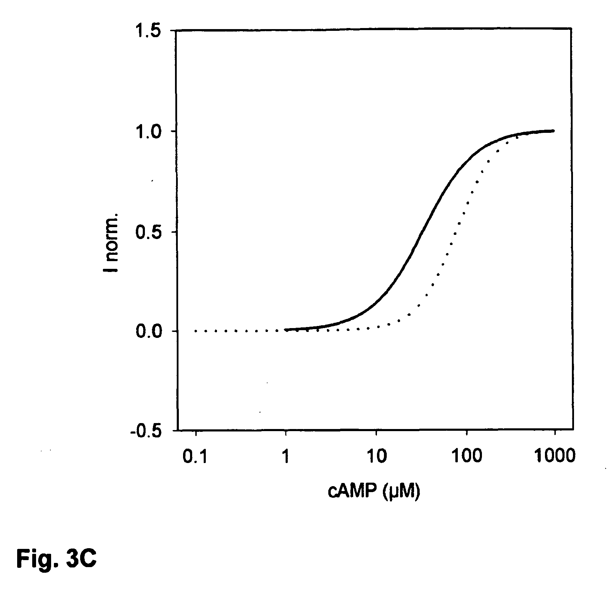

Die T537M-Mutante wird durch 2,7 µM cAMP (Abb. 1C), die T537V-Mutante durch 34 µM cAMP (Abb. 3C) und der Wildtyp durch 80 µM cAMP (Abb. 1C, 3C) halbmaximal aktiviert. Im Vergleich zum Wildtyp ist die T537M-Mutante etwa um den Faktor 30 und die T537V-Mutante etwa um den Faktor 3 sensitiver für cAMP. Im Vergleich zur T537S-Mutante ist die T537M-Mutante etwa um den Faktor 5 sensitiver für cAMP, während die T537V-Mutante etwa um den Faktor 2,5 weniger sensitiv für cAMP ist als die T537S-Mutante.

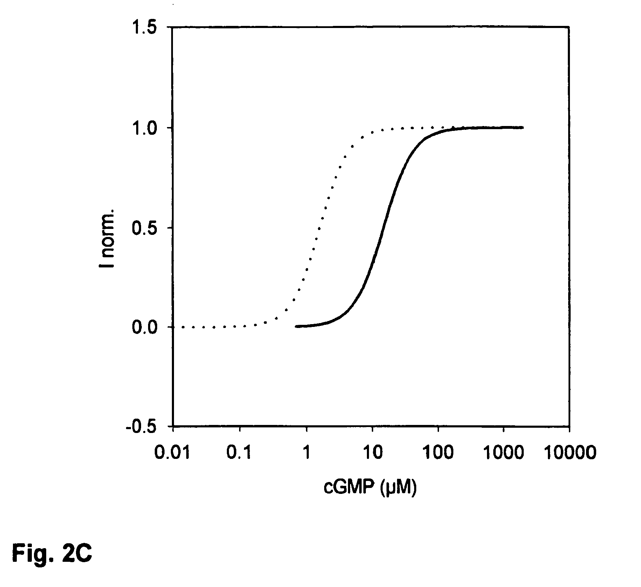

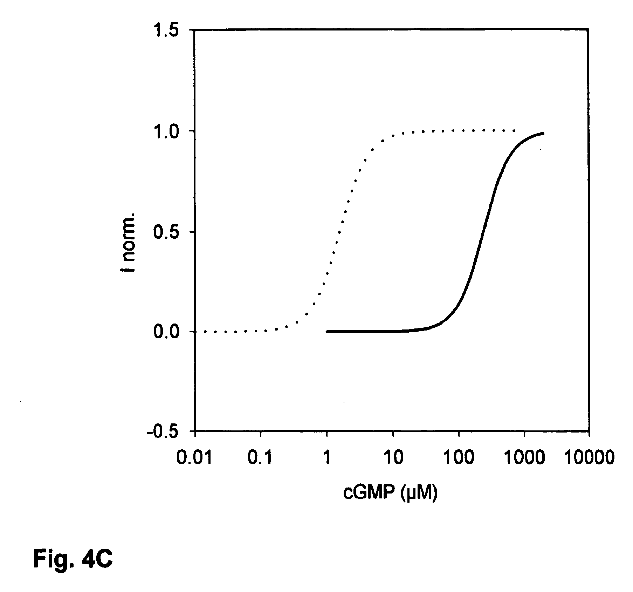

Die T537M-Mutante wird durch 14,9 µM cGMP (Abb. 2C), die T537V-Mutante (Abb. 4C) durch 241 µM cGMP und der Wildtyp durch 1,6 µM cGMP (Abb. 2C, 4C) halbmaximal aktiviert. Im Vergleich zum Wildtyp ist die T537M-Mutante also etwa um den Faktor 9 und die T537V-Mutante etwa um den Faktor 150 weniger sensitiv für cGMP. Im Vergleich zur T537S-Mutante ist die T537M-Mutante um den Faktor 21 und die T537V-Mutante sogar etwa um den Faktor 350 weniger sensitiv für cGMP.

Die Selektivität eines CNG-Kanals für cAMP bzw. cGMP ergibt sich aus dem Vergleich der K_{1/2}-Werte. Beispiel: Die T537M-Mutante besitzt einen K_{1/2}-Wert von 2,7 µM für cAMP und einen K_{1/2}-Wert für cGMP von 14,9 µM. Dies bedeutet, daß die Mutante bereits durch 2,7 µM cAMP, aber erst durch 14,9 µM cGMP halbmaximal aktiviert wird. Die Mutante ist demnach deutlich sensitiver für cAMP als für cGMP. Der Quotient (14,9µM/2,7µM) gibt die relative Selektivität an. Die T537M-Mutante besitzt also eine etwa 6 fach höhere Selektivität für cAMP. Die T537V-Mutante ist ebenfalls etwa 6 fach selektiver für cAMP. Die T537S-Mutante hingegen besitzt eine 20 fach und der Wildtyp-Kanal sogar eine etwa 50 fach höhere Selektivität für cGMP.

Die Ergebnisse der Messungen zeigen folgendes: Es wurden CNG-Kanalmutanten erzeugt und identifiziert, die (1) eine sehr viel größere absolute cAMP-Empfindlichkeit aufweisen als die Wildtyp-Kanäle, und (2) wird die Selektivität für cAMP und cGMP umgedreht. Die genetischen Modifikationen verleihen diesen CNG-Kanälen eine enorme selektive Sensitivität für cAMP. Zusätzlich sind die beiden genetisch modifizierten CNG-Kanäle so unempfindlich für cGMP, daß selbst größere Änderungen der intrazellulären cGMP-Konzentration die Messung der cAMP-Konzentration nicht stören. Insbesondere die erfindungsgemäß bevorzugte T537M-Mutante eignet sich besonders gut als zellulärer cAMP-Sensor. Sie kann beispielsweise in zellulären Testsystemen zur Untersuchung der Wirkung von Substanzen eingesetzt werden, die die intrazelluläre cAMP-Konzentration beeinflussen können, und macht solche Testsysteme erst praktisch nutzbar: Mit einem solchen Sensor können dann auch solche geringfügige Änderungen der intrazellulären cAMP-Konzentration, wie sie beispielsweise durch die Aktivierung von GPCRs ausgelöst werden, zuverlässig mit einem sehr guten Signal/Rausch-Verhältnis registriert und in Echtzeit verfolgt werden.

### Methoden

### Herstellung genetisch motivierter CNG-Kanäle durch zielgerichtete Mutagenese

Die cDNA für die α3-Untereinheit des CNG-Kanals aus Rind wurde mit Hilfe geeigneter Restriktionsendonukleasen (Eco RV und Nsi I) aus dem Plasmid pCHOLF102 (Altenhofen W, Ludwig J, Eismann E, Kraus W, Bönigk W, and Kaupp UB. (1991) Control of ligand specificity in cyclic nucleotide-gated channels from rod photoreceptors and olfactory epithelium. Proc Natl Acad Sci USA, 88, 9868-9872) ausgeschnitten und in pcD-NAlamp (Invitrogen) kloniert. Das Plasmid wurde als pcA-bolf bezeichnet. Das cDNA-Fragment wurde dann über Eco RV und Xba I in pcDNA3-Derivate kloniert. Das Plasmid wurde als pc3-bolf bezeichnet.
Die Herstellung der genetisch modifizierten α3-Untereinheit des CNG-Kanals erfolgte mittels zielgerichteter Mutagenese (Herlitze S and Koenen M (1990). A general and rapid mutagenisis method using polymerase chain reaction. Gene, 91, 143-147).

Der Mutagensese-Primer zur Herstellung der T537M-α3-Untereinheit hatte folgende Sequenz (SEQ ID NO 7):
5'- CGACGCATGGCGAACATCCGCAGTCT-3'
Der Mutagenese-Primer zur Herstellung der T537V-α3-Untereinheit hatte folgende Sequenz (SEQ ID NO 8): 5'- CGACGCGTCGCGAACATCCG-CAGTCT-3'

Zunächst wurde eine PCR mit dem Mutagenese-Primer und dem Gegenprimer #1817 (5'- TTGGCTGCAGCTATTATGGCTTCTCGGCAG -3') (SEQ ID NO 9) auf pc3-bolf durchgeführt. Ein 100 µl PCR-Ansatz enthielt 10 ng Matrizen-DNA, 1 x PCR-Puffer für Taq-Polymerase incl. 1,5 mM MgCl₂, 200 µM dNTPs, 1 U Taq-Polymerase und jeweils 150 ng der beiden Primer. Die PCR-Bedingungen waren wie folgt. 2 Minuten Denaturierung bei 94 °C, gefolgt von 25 Zyklen ä 45 sec 94°C, 45 sec 46°C, 45 sec 72°C. Das 404 Bp große Fragment wurde gereinigt und zusammen mit dem überlappenden 666 Bp großem Sma I / Pvu II Restriktionsfragment als Matrize für eine weitere PCR eingesetzt. Zur Amplifikation wurden die flankierenden Primer #1813 (5'- GTCGGATCCTCCACACTCAA-GAAAGTG -3') (SEQ ID NO 10) und #1817 eingesetzt. Der PCR-Ansatz hatte die gleiche Zusammensetzung wie oben angegeben, wobei statt 10 ng Plasmid-DNA als Matrize 250 ng des 1. PCR-Fragments und 10 ng des Restriktionsfragments eingesetzt wurden. Das PCR-Fragment wurde mit BsrGl geschnitten und gegen das entsprechende Fragment in pc3-bolf ausgetauscht. Die Sequenz des veränderten DNA-Abschnitts wurde durch Sequenzierung überprüft.

### Isolierung von cDNA-Klonen

Für die Klonierung des CRF-Rezeptors wurde eine PCR mit den Primern #843 (5'-AGCGGGATCCACCATGGGACGGCGCCCGCA-3') (SEQ ID NO 11) und #842 (5'- GGCCTGGAGCTCACACTG -3') (SEQ ID NO 12) auf Erststrang-cDNA von Ratten-Hypophysen durchgeführt. Ein 100 µl PCR-Ansatz enthielt 10 ng Erststrang-cDNA, 1 x PCR-Puffer für Taq-Polymerase incl. 1,5 mM MgCl₂, 200 µM dNTPs, 1 U Taq-Polymerase und jeweils 150 ng der beiden Primer. Die PCR-Bedingungen waren wie folgt. 2 Minuten Denaturierung bei 94 °C, gefolgt von 44 Zyklen à 45 sec 94°C, 45 sec 56°C, 75 sec 72°C. Das 1271 Bp große Fragment wurde über BamHI und Sacl in pBluescript SK⁻ kloniert (pBCRFR1). Die Sequenz stimmt mit der veröffentlichen Sequenz L25438 (Chang, CP, Pearse RVII, O'Connel S, and Rosenfed MG (1993). Identification of a seven transmembrane helix receptor for corticotropin-releasing factor and sauvagine in mammalian brain. Neuron, 11, 1187-1195) überein. Für die heterologe Expression wurde die cDNA in pcDNA-Derivate umkloniert (pcCRFR1).

Das Plasmid pcK₁M, das die cDNA des Dopamin-Rezeptor aus Drosophila enthält (Gotzes F, Balfanz S, and Baumann A. (1994) Primary structure and functional characterization of a Drosophila dopamine receptor with high homology to human D1/5 receptors. Receptors Channels, 2, 131-141), wurde freundlicherweise von Dr. F. Gotzes zur Verfügung gestellt.

### Heterologe Expression in HEK 293-Zellen und Herstellung der stabilen Zelllinien

Die transiente Expression in HEK 293-Zellen erfolgte wie in Baumann A, Frings S, Godde M, Seifert R, and Kaupp UB. (1994) Primary structure and functional expression of a Drosophila cyclic nucleotide-gated channel present in eyes and antennae. EMBO J., 13, 5040-5050, beschrieben. Zur elektrophysiologischen Charakterisierung wurden die Zellen am Tag nach der Transfektion auf Glasplättchen umgesetzt, die mit Poly-L-Lysin beschichtet worden waren. Die elektrophysiologischen Untersuchungen wurden dann am darauffolgenden Tag durchgeführt.
Für die Herstellung der stabilen Zelllinien wurden die Zellen in der gleichen Weise transfiziert. Zur Selektion von Zellklonen, die das gewünschte Gen stabil exprimierten, wurden 2x10⁴ Zellen am Tag nach der Transfektion auf eine 9 cm Zellkulturschale ausgesät. Die Zellen wurden 20 Tage kultiviert, wobei dem Zellkultur-Medium entweder G418 (800 µg/ml) (Calbiochem), Zeocin (100 µg/ml) (Invitrogen) oder Hygromycin (100 µg/ml) (Invitrogen) zugesetzt wurden. Nach 20 Tagen wurden die Zellklone, die das Resistenzgen exprimierten, vereinzelt und die Expression des CNG-Kanalgen bzw. des Rezeptorgens durch Westernblot-Analysen und funktionelle Untersuchungen (elektrophysiologische Messungen und fluoreszenz-optische Messungen) überprüft. Für die Westernblot-Analysen wurde die Zellen in einem Lysepuffer homogenisiert (10 mM Hepes, 1 mM DTT und 1 mM ETDA bei pH 7,4), 5 x schockgefroren (in flüssigem Stickstoff) und schließlich 10 min bei 55 000 Upm zentrifugiert. Das Membranpellet wurde in Lösungspuffer resuspendiert (150 mM NaCl, 1 mM MgCl₂, 20 mM Hepes bei pH 7,5, 0,1 mM EGTA und 0,5 % Triton X-100). Jeweils 3 µg der Membranproteine wurden mittels SDS-PAGE getrennt, auf Immobilon-Membranen übertragen und mit spezifischen Antikörpern markiert. Die Immunoreaktivität wurde mit Hilfe des ECL-Detektionskits (Amersham) sichtbar gemacht.

Zur Herstellung der doppelt-stabilen Zelllinien wurde ein Zellklon, der entweder das CNG-Kanalgen oder das Rezeptorgen stabil exprimierte, weiter kultiviert und zur Transfektion mit der jeweils anderen cDNA eingesetzt. Die Selektion und Auswahl der Zellklone erfolgte wie oben beschrieben.

Für die Fluoreszenz-optischen Messungen wurden zum Teil Zellen verwendet, die eine genetisch-modifizierte α3-Untereinheit des CNG-Kanals stabil und den Dopamin-Rezeptor aus Drosophila transient exprimierten. Dazu wurden Zellen der stabilen T537M-Zelllinie, wie von Baumann A, Frings S, Godde M, Seifert R, and Kaupp UB. (1994) Primary structure and functional expression of a Drosophila cyclic nucleotide-gated channel present in eyes and antennae. EMBO J., 13, 5040-5050 beschrieben, transfiziert. Am Tag nach der Transfektion wurden die Zellen in eine Multiwell-Platte mit 96 Vertiefungen ausgesät. Die Zelldichte pro well betrug 2x10⁴ Zellen.
Für die Fluoreszenz-optischen Messungen von stabilen Zelllinien wurden die Zellen ein bis zwei Tage vor der Messung in die Multiwell-Platte mit 96 Vertiefungen ausgesät. Die Zelldichte betrug 1,5 bis 4 x 10⁴ Zellen.

### Elektrophysiologie

Die genetisch modifizierten und die Wildtyp-CNG-Kanäle wurden jeweils heterolog in HEK 293-Zellen exprimiert (Baumann A, Frings S, Godde M, Seifert R, and Kaupp UB. (1994) Primary structure and functional expression of a Drosophila cyclic nucleotide-gated channel present in eyes and antennae. EMBO J., 13, 5040-5050). Die elektrophysiologische Charakterisierung der genetisch modifizierten CNG-Kanäle erfolgte mit der Patch-Clamp-Technik unter Spannungsklemmbedingungen. Die Aktivierungseigenschaften der Kanäle wurden bestimmt und mit denen des Wildtyp-Kanals verglichen (Abbildungen 1 bis 4):

Von Zellen, die genetisch modifizierte oder Wildtyp-CNG-Kanäle stabil exprimieren, wurden inside-out Patches exzidiert. Die Membranpatches wurden mit verschiedenen Konzentrationen an zyklischen Nukleotiden in der Badlösung umspült. Ausgehend von einer Haltespannung von 0 mV wurden Spannungssprünge zu verschiedenen Testspannungen zwischen -100 mV und +100 mV bei einer Schrittweite von 20 mV am Patch appliziert. Die Ströme wurden mit Standardmethoden registriert und analysiert.

Abbildung 1A zeigt die Strom-Spannungs- (IV-) Beziehung der zeigt T537M-α3-Untereinheit des CNG-Kanals aus Rind. Es sind die mittleren Ströme (I) bei verschiedenen Konzentrationen von cAMP gegen die Spannung (Vm) aufgetragen: 0 µM (gefüllte Kreise), 0,3 µM (offene Kreise), 1 µM (gefüllte Dreiecke), 3 µM (offene Dreiecke), 10 µM (gefüllte Quadrate), 30 µm (offene Quadrate), 100 µM (gefüllte Karos).

Abbildung 1 B zeigt die Abhängigkeit der mittleren Ströme von der cAMP-Konzentration für die T537M-α3-Untereinheit des CNG-Kanals aus Rind bei einer Spannung von +100 mV (gefüllte Kreise). Die durchgezogene Linie ist nach der Hillgleichung I = (Iₘₐₓ - Iₘᵢₙ) cⁿ / (Kⁿ+cⁿ) + Iₘᵢₙ (I: Strom; Iₘₐₓ: maximaler Strom; Iₘᵢₙ: minimaler Strom; K_{1/2}: Konzentration, bei der die Kanäle halbmaximal aktiviert sind; n: Hillkoeffizient; c: cAMP-Konzentration) mit folgenden Parametern berechnet worden: Iₘₐₓ = 328 pA; Imin = 24 pA; K_{1/2}= 2,9 µM; n = 2,4.

Abbildung C zeigt die normierte Dosis-Wirkungsbeziehung der T537M-α3-Untereinheit des CNG-Kanals aus Rind (durchgezogene Linie) und die der Wildtyp α3-Untereinheit aus Rind (gepunktete Linie) bei +100 mV. Die durchgezogene sowie die gepunktete Linie sind nach der normierten Hillgleichung Iₙₒᵣₘ = cⁿ /(cⁿ + Kⁿ) mit folgenden gemittelten Parametern berechnet worden: (durchgezogene Linie: K_{1/2} = 2,7 µM; n = 2,4; gepunktete Linie: K_{1/2} = 80 µM; n = 2,0).

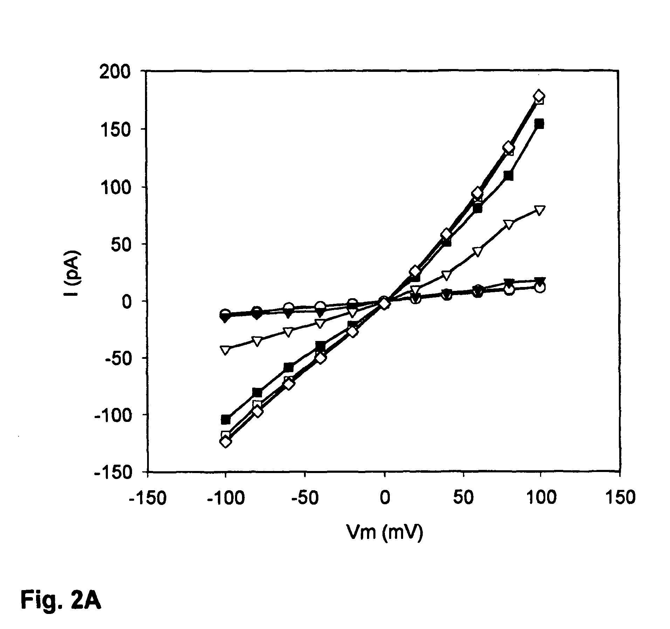

Abbildung 2A zeigt die Strom-Spannungs- (IV-) Beziehung der heterolog exprimierten T537M-α3-Untereinheit des CNG-Kanals aus Rind. Es sind die mittleren Ströme (I) bei verschiedenen Konzentrationen von cGMP gegen die Spannung (Vm) aufgetragen: 0 µM (gefüllte Kreise), 1 µM (offene Kreise), 3 µM (gefüllte Dreiecke), 10 µM (offene Dreiecke), 30 µM (gefüllte Quadrate), 100 µM (offene Quadrate), 300 µM (gefüllte Karos), 2000 µM (offene Karos).

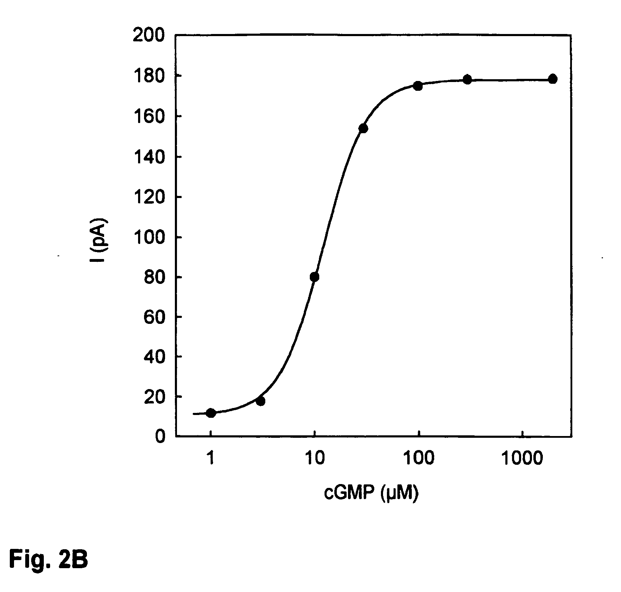

Abbildung 2B zeigt die Abhängigkeit der mittleren Ströme von der cGMP-Konzentration für die T537M-α3-Untereinheit des CNG-Kanals aus Rind bei einer Spannung von +100 mV (gefüllte Kreise). Die durchgezogene Linie ist nach der Hillgleichung I = (Iₘₐₓ - Iₘᵢₙ) cⁿ / (K"+c") + Iₘᵢₙ (I: Strom; Iₘₐₓ: maximaler Strom; Iₘᵢₙ: minimaler Strom; K_{1/2}: Konzentration, bei der die Kanäle halbmaximal aktiviert sind; n: Hillkoeffizient; c: cGMP-Konzentration) mit folgenden Parametern berechnet worden: Iₘₐₓ = 167 pA; Iₘᵢₙ = 11 pA; K_{1/2} = 12,0 µM; n = 2,0.

Abbildung 2C zeigt die normierte Dosis-Wirkungsbeziehung von der T537M-α3-Untereinheit des CNG-Kanals aus Rind (durchgezogene Linie) und die der Wildtyp-α3-Untereinheit aus Rind (gepunktete Linie) bei +100 mV. Die durchgezogene sowie die gepunktete Linie sind nach der normierten Hillgleichung I_{norm.} = cⁿ /(cⁿ + Kⁿ) mit folgenden gemittelten Parametern berechnet worden: (durchgezogene Linie: K_{1/2}= 14,9 µM; n = 1,9; gepunktete Linie: K_{1/2} = 1,6 µM; n = 2,0).

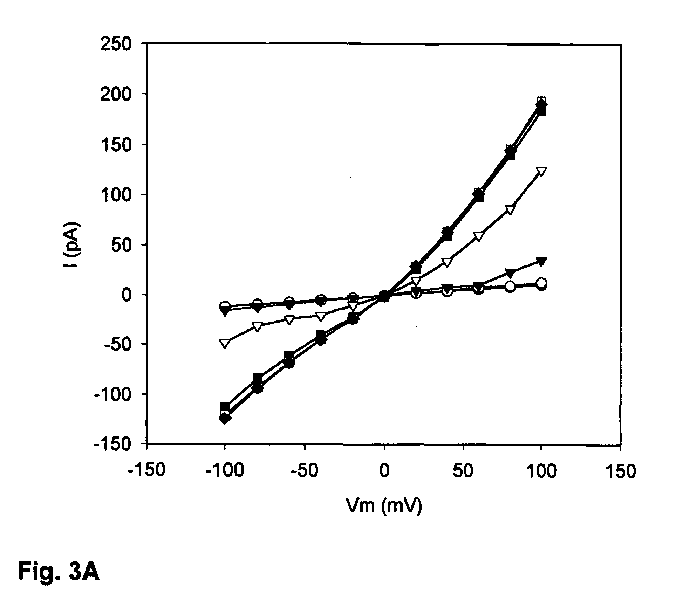

Abbildung 3A zeigt die Strom-Spannungs- (IV-) Beziehung der heterolog exprimierten T537V-α3-Untereinheit des CNG-Kanals aus Rind. Es sind die mittleren Ströme (I) bei verschiedenen Konzentrationen von cAMP gegen die Spannung (Vm) aufgetragen: 0 µM (gefüllte Kreise), 3 µM (offene Kreise), 10 µM (gefüllte Dreiecke), 30 µM (offene Dreiecke), 100 µM (gefüllte Quadrate), 300 µM (offene Quadrate), 1000 µM (gefüllte Karos).

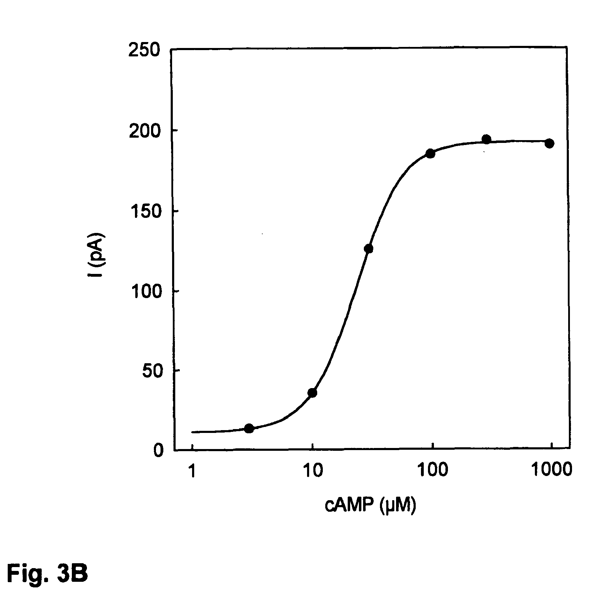

Abbildung 3B zeigt die Abhängigkeit der mittleren Ströme von der cAMP-Konzentration für die T537V-α3-Untereinheit des CNG-Kanals aus Rind bei einer Spannung von +100 mV (gefüllte Kreise). Die durchgezogene Linie ist nach der Hillgleichung I = (Iₘₐₓ - Iₘᵢₙ) cⁿ / (Kⁿ+cⁿ) + Imin (I: Strom; Iₘₐₓ: maximaler Strom; Iₘᵢₙ: minimaler Strom; K_{1/2}: Konzentration, bei der die Kanäle halbmaximal aktiviert sind; n: Hillkoeffizient; c: cAMP-Konzentration) mit folgenden Parametern berechnet worden:
Iₘₐₓ= 181 pA; Iₘᵢₙ = 11 pA; K_{1/2} = 23,4 µM; n = 2,2.

Abbildung 3C zeigt die normierte Dosis-Wirkungsbeziehung der T537V-α3-Untereinheit des CNG-Kanals aus Rind (durchgezogene Linie) und die der Wildtyp-α3-Untereinheit aus Rind (gepunktete Linie) bei +100 mV. Die durchgezogene sowie die gepunktete Linie sind nach der normierten Hillgleichung I_{norm.} = cⁿ /(cⁿ + Kⁿ) mit folgenden gemittelten Parametern berechnet worden: (durchgezogene Linie: K = 34 µM; n = 1,5; gepunktete Linie: K_{1/2} = 80 µM; n = 2,0).

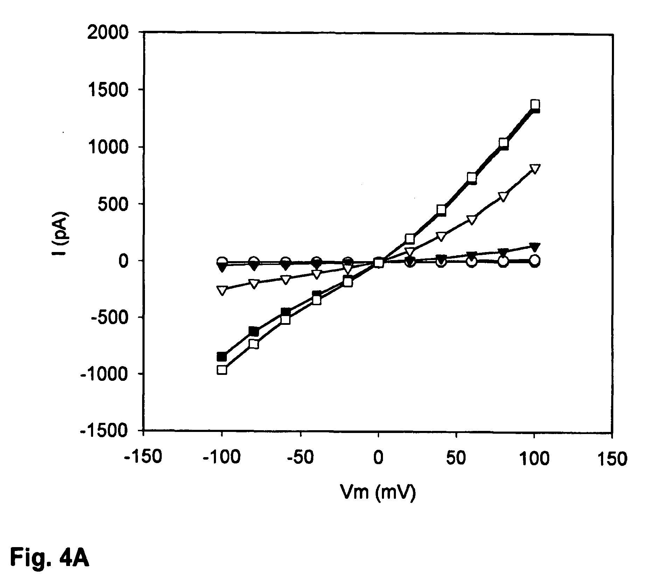

Abbildung 4A zeigt die Strom-Spannungs- (IV-) Beziehung der heterolog exprimierten T537V-α3-Untereinheit des CNG-Kanals aus Rind. Es sind die mittleren Ströme (I) bei verschiedenen Konzentrationen von cGMP gegen die Spannung (Vm) aufgetragen: 0 µM (gefüllte Kreise), 30 µM (offene Kreise), 100 µM (gefüllte Dreiecke), 300 µM (offene Dreiecke), 1000 µM (gefüllte Quadrate), 2000 µM (offene Quadrate).

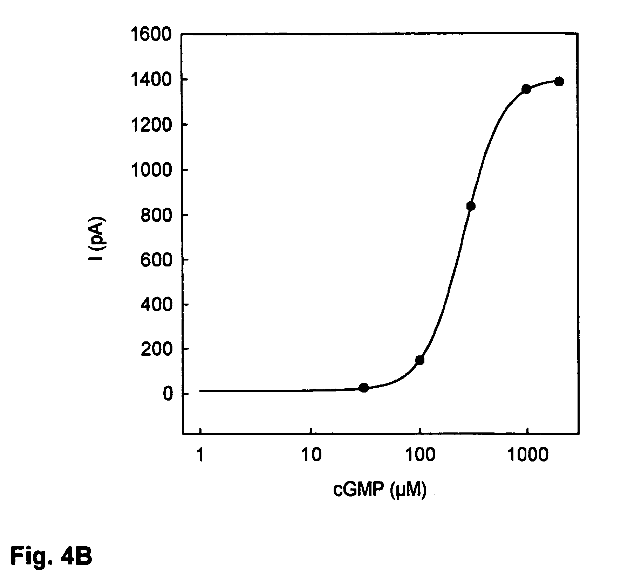

Abbildung 4B zeigt die Abhängigkeit der mittleren Ströme von der cGMP-Konzentration für die T537V-α3-Untereinheit des CNG-Kanals aus Rind bei einer Spannung von +100 mV (gefüllte Kreise). Die durchgezogene Linie ist nach der Hillgleichung I = (Iₘₐₓ - Iₘᵢₙ) cⁿ / (Kⁿ+cⁿ) + Iₘᵢₙ (I: Strom; Iₘₐₓ: maximaler Strom; I_{min:} minimaler Strom; K_{1/2}: Konzentration bei der die Kanäle halbmaximal aktiviert sind; n: Hillkoeffizient; c: cGMP-Konzentration) mit folgenden Parametern berechnet worden: Iₘₐₓ = 1389 pA; Iₘᵢₙ = 13 pA; K_{1/2} = 255,2 µM; n = 2,2.

Abbildung 4C zeigt die normierte Dosis-Wirkungsbeziehung der T537V-α3-Untereinheit aus Rind (durchgezogene Linie) und der Wildtyp-a3-Untereinheit aus Rind (gepunktete Linie) bei +100 mV. Die durchgezogene sowie die gepunktete Linie sind nach der normierten Hillgleichung Iₙₒᵣₘ. = cⁿ /(cⁿ + Kⁿ) mit folgenden gemittelten Parametern berechnet worden: (durchgezogene Linie: K = 241 µM; n = 2,1; gepunktete Linie: K_{1/2} = 1,6 µM; n = 2,0).

### Fluoreszenz-optische Messungen

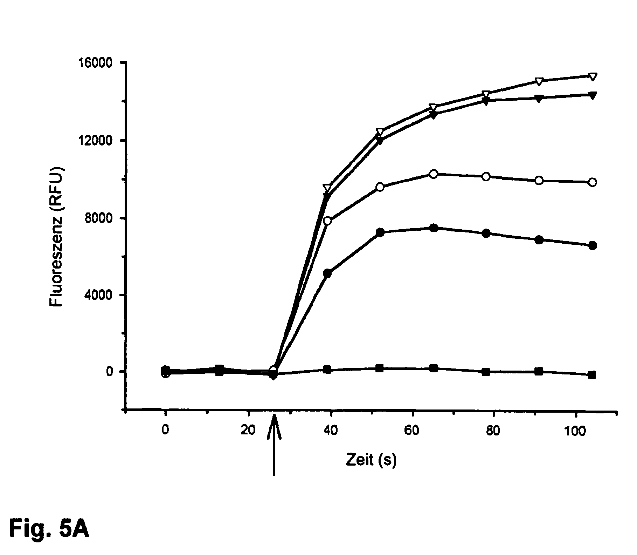

Die fluorimetrischen Messungen der intrazellulären Ca²⁺-Konzentration wurden in Multiwell-Platten mit 96 Vertiefungen durchgeführt (Abb. 5A-5G). Die Zellen wurden vor der Messung eine Stunde mit dem Ca²⁺-sensitivem Fluoreszenzfarbstoff Fluo-4 beladen. Die Beladungslösung enthielt 120 mM NaCl, 3 mM KCI, 50 mM Glucose, 10 mM Hepes (pH 7,4), 3 mM MgCl₂ und 4 µM Fluo4-AM (Molecular Probes). Die Messlösung enthielt 120 mM NaCl, 3 mM KCI, 50 mM Glucose, 10 mM Hepes (pH 7,4) und 3 mM CaCl₂. Der zeitliche Verlauf der Änderung der Fluoreszenzintensität nach Applikation von Agonisten, Antagonisten, Enzym-Aktivatoren oder Enzym-Inhibitoren wurde mit einem Fluoreszenzreader (FLUOstar, BM-G-Labtechnologies) registriert. Die Anregungswellenlänge betrug 485 nm. Die Emissionswellenlänge betrug 520 nm.

### Beispiel 5

Die Abbildung 5A zeigt die fluorimetrisch gemessene Änderung der intrazellulären Ca²⁺-Konzentration in Zellen, die den Dopamin-Rezeptor (DR) aus Drosophila (Gotzes F, Balfanz S, and Baumann A. (1994) Primary structure and functional characterization of a Drosophila dopamine receptor with high homology to human D1/5 receptors. Receptors Channels, 2, 131-141) transient und die T537M-Mutante des α3-CNG-Kanals stabil exprimieren. Dargestellt ist der zeitliche Verlauf der Fluoreszenzintensität (RFU: relative Fluoreszenzunit) nach Stimulation der Zellen mit unterschiedlichen Konzentrationen des Agonisten Dopamin. Der Pfeil markiert den Zeitpunkt, an dem die Zellen mit Dopamin stimuliert wurden. Die Dopamin-induzierte Aktivierung des Dopamin-Rezeptors führt in der Zelle zunächst zur Aktivierung eines stimulatorischen G-Proteins und schließlich zur Aktivierung einer Adenylatzyklase, die cAMP synthetisiert. Durch die Bindung von cAMP werden CNG-Kanäle geöffnet und Ca²⁺-lonen strömen in die Zelle. Die Schnelligkeit und das Ausmaß der Änderung der Ca²⁺-Konzentration hängt von der Dopamin-Konzentration ab. Die Dopamin-Konzentration betrug 25 nM (gefüllte Kreise), 50 nM (offene Kreise), 400 nM (gefüllte Dreiecke) oder 600 nM (offene Dreiecke). Zum Vergleich wurden auch Zellen, die den Dopamin-Rezeptor nicht exprimieren, mit 25 nM Dopamin stimuliert (gefüllte Vierecke).

### Beispiel 6

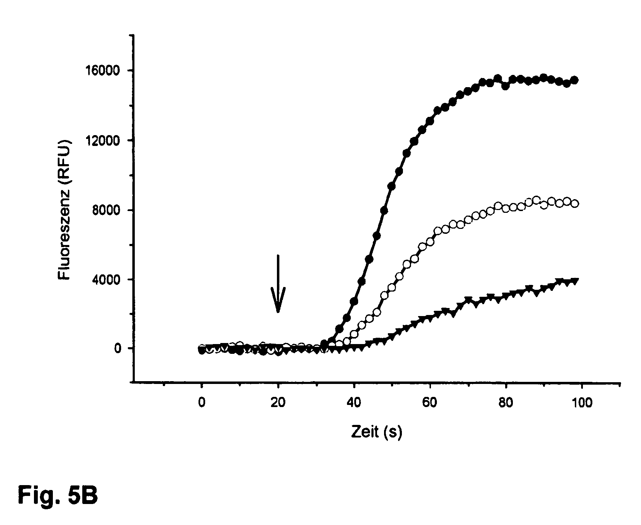

Die Abbildung 5B zeigt die fluorimetrisch gemessene Änderung der intrazellulären Ca²⁺-Konzentration in Zellen, die sowohl den Corticotropin-Releasing-Factor (CRF)-Rezeptor als auch die T537M-Mutante des α3-CNG-Kanals stabil exprimieren. Dargestellt ist der zeitliche Verlauf der Fluoreszenzintensität nach Stimulation der Zellen mit dem Agonisten CRF. Der Pfeil markiert den Zeitpunkt, an dem die Zellen mit CRF stimuliert wurden.

Die CRF-induzierte Aktivierung des CRF-Rezeptors führt in der Zelle zunächst zur Aktivierung eines stimulatorischen G-Proteins und schließlich zur Aktivierung der Adenylatzyklase, die cAMP synthetisiert (siehe z.B.: Eckart K, Radulovic J, Radulovic M, Jahn O, Blank T, Stiedl O, and Spiess J. (1999) Actions of CRF and its analogs. Curr Med Chem., 6, 1035-1053; Perrin MH and Vale WW (1999). Corticotropin releasing factor receptors and their ligand family. Ann. N.Y. Acad. Sci., 885, 312-328). Durch die Bindung von cAMP werden CNG-Kanäle geöffnet und Ca²⁺-Ionen strömen in die Zelle. Die Schnelligkeit und das Ausmaß der Ca²⁺-Änderung hängt von der CRF-Konzentration ab. Die CRF Konzentration betrug 100 pM (gefüllte Dreiecke), 300 pM (offene Kreise) oder 1000 pM (gefüllte Kreise).

Die Abbildungen 5A und 5B zeigen beispielhaft für zwei unterschiedliche GCRPs, die an ein stimulatorisches G-Protein koppeln, daß mit dem erfindungsgemässen Verfahren die Wirkung von Agonisten auf diese GCRPs mit großer Empfindlichkeit gemessen werden kann. Das Verfahren eignet sich gleichermaßen für alle anderen GCRPs, die an stimulatorische G-Proteine koppeln. Die Beispiele aus Abbildung 5A und 5B zeigen auch, daß für das erfindungsgemässe Verfahren die heterolog exprimierten Proteine (genetisch modifizierte α-Untereinheit des CNG-Kanals und GCRP) sowohl transient, als auch stabil in den Zellen exprimiert werden können.

### Beispiel 7

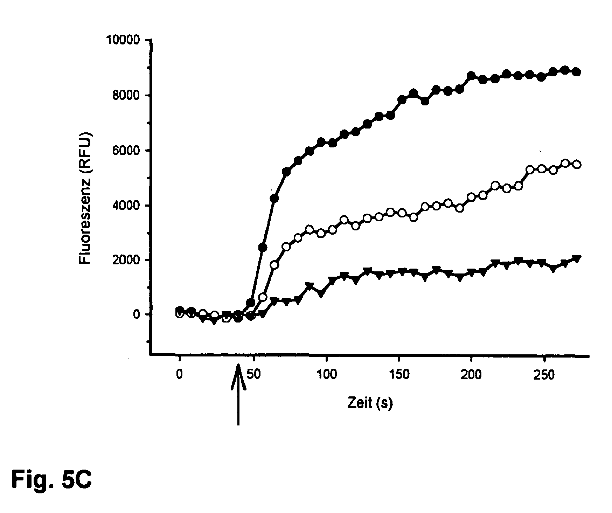

Die Abbildung 5C zeigt, daß das Verfahren geeignet ist, um die Wirksamkeit verschiedener Antagonisten quantitativ zu bestimmen. Zellen, die den Corticotropin-Releasing-Factor (CRF)-Rezeptor und die T537M-Mutanten des α3-CNG-Kanals stabil exprimieren, wurden mit dem helikalen Antagonisten 9-41 (Rivier J, Rivier C, and Vale WW. (1984) Synthetic competitive antagonists of corticotropin-releasing factor: effect on ACTH secretion in the rat. Science, 224, 889-891) behandelt, bevor sie mit CRF (1 nM) stimuliert wurden. Dargestellt ist der zeitliche Verlauf der Fluoreszenzintensität. Der Pfeil markiert den Zeitpunkt, an dem die Zellen mit CRF stimuliert wurden. Bei gleicher CRF-Konzentration hängt die Schnelligkeit und das Ausmaß der Ca²⁺-Änderung von der Konzentration des Antagonisten ab. Die Konzentration des Antagonisten betrug 0 nM (gefüllte Kreise), 10 nM (offene Kreise) oder 100 nM (gefüllte Dreiecke).

### Beispiel 8

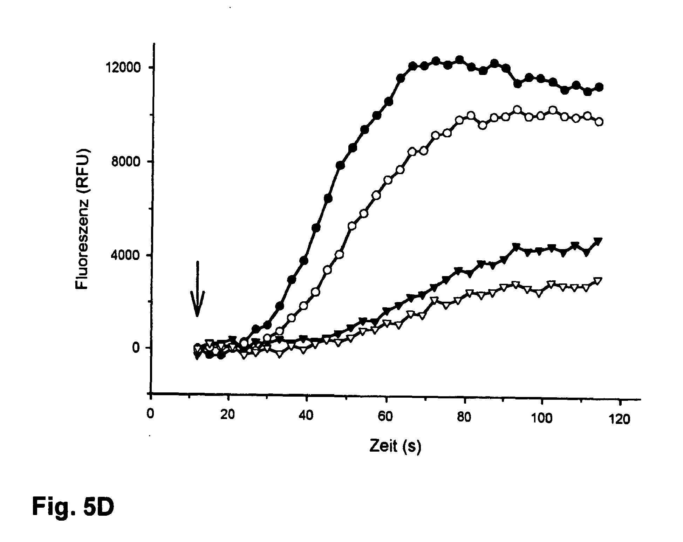

Die Abbildung 5D zeigt, daß das Verfahren geeignet ist, die Wirksamkeit von bestimmten Enzym-Aktivatoren zu messen. Dargestellt ist der Effekt eines Aktivators der Adenylatzyklase (AC) auf die Änderung der intrazellulären Ca²⁺-Konzentration. Zellen, die die T537M-Mutanten des α3-CNG-Kanals stabil exprimieren, wurden mit unterschiedlichen Konzentrationen des AC-Aktivators Forskolin stimuliert. Dargestellt ist der zeitliche Verlauf der Fluoreszenzintensität. Der Pfeil markiert den Zeitpunkt, an dem die Zellen mit Forskolin stimuliert wurden. Forskolin aktiviert direkt die endogene Adenylatzyklase der Zellen (Seamon, KB and Daly, JW. (1981) Forskolin: a unique diterpene activator of cyclic AMP-generating systems. J Cyclic Nucleotide Res., 7, 201-224).
Die Geschwindigkeit und das Ausmaß der Änderung der intrazellulären Ca²⁺-Konzentration hängt von der Konzentration des AC-Aktivators ab. Die Konzentration von Forskolin betrug 0,5 µM (offene Dreiecke), 0,75 µM (gefüllte Dreiecke), 2 µM (offene Kreise) oder 4 µM (gefüllte Kreise).

### Beispiel 9

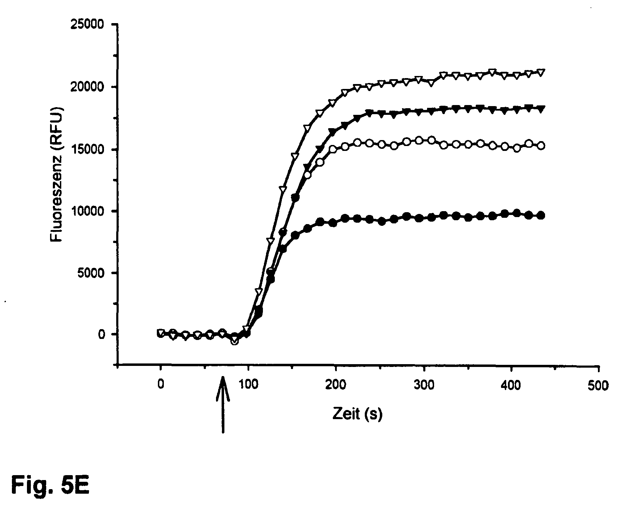

Abbildung 5E zeigt, daß das Verfahren sich eignet, um die Wirksamkeit von bestimmten Enzym-Inhibitoren zu bestimmen. Dargestellt ist der Effekt eines Phosphodiesterase (PDE)-Inhibitors auf die Forskolin-induzierte Änderung der intrazellulären Ca²⁺-Konzentration. Zellen, die die T537M-Mutanten des α3-CNG-Kanals stabil exprimieren, wurden zunächst mit unterschiedlichen Konzentrationen des PDE-Inhibitors IBMX behandelt und dann mit 5 µM Forskolin stimuliert. Aufgetragen ist der zeitliche Verlauf der Fluoreszenzintensität. Der Pfeil markiert den Zeitpunkt, an dem die Zellen mit Forskolin stimuliert wurden. Der PDE-Inhibitor hemmt die Aktivität der endogenen PDEs und vermindert dadurch den Abbau von cAMP, das durch die Stimulierung der endogenen AC synthetisiert wurde.

Das Ausmaß der Änderung der intrazellulären Ca²⁺-Konzentration hängt von der Konzentration des PDE-Inhibitors ab. Die IBMX-Konzentration betrug 0 µM (gefüllte Kreise), 10 µM (offene Kreise), 50 µM (gefüllte Dreiecke) oder 100 µM (offene Dreiecke).

Das Beispiel zeigt, daß das Verfahren geeignet ist, um die Wirksamkeit von PDE-Inhibitoren empfindlich zu messen. Es können Inhibitoren von endogenen PDEs, aber auch Inhibitoren von heterolog exprimierten PDEs untersucht werden.

Das efindungsgemässe Verfahren eignet sich auch zur Bestimmung der Aktivität von GCRPs, die an das inhibitorische G-Protein Gᵢ koppeln. Werden solche GCRPs aktiviert, so sinkt die cAMP-Konzentration in der Zelle ab, weil ACs inhibiert werden. Für das erfindungsgemäße Verfahren müssen die Zellen zunächst zur cAMP-Synthese stimuliert werden (z.B. durch den AC-Aktivator Forskolin), bevor dann die Aktivität von Agonisten gemessen werden kann. Durch gleichzeitige oder sequentielle Applikation von Antagonisten und Agonisten kann auch die Wirksamkeit von Antagonisten für solche GCRP getestet werden.
Das Verfahren eignet sich in analoger Weise für die Bestimmung sämtlicher Substanzen, die in irgendeiner Weise die cAMP-Konzentration in der Zelle erhöhen oder erniedrigen können. Die direkten Angriffspunkte dieser Substanzen (z.B. GCRP oder Enzyme) können entweder endogen in den Zellen vorhanden sein oder transient oder stabil in Zellen zusammen mit genetisch modifizierten CNG-Kanälen exprimiert werden.

### Beispiel 10

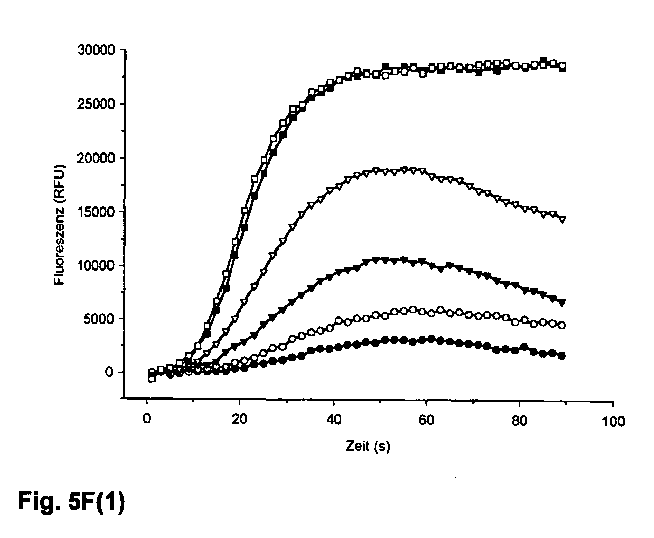

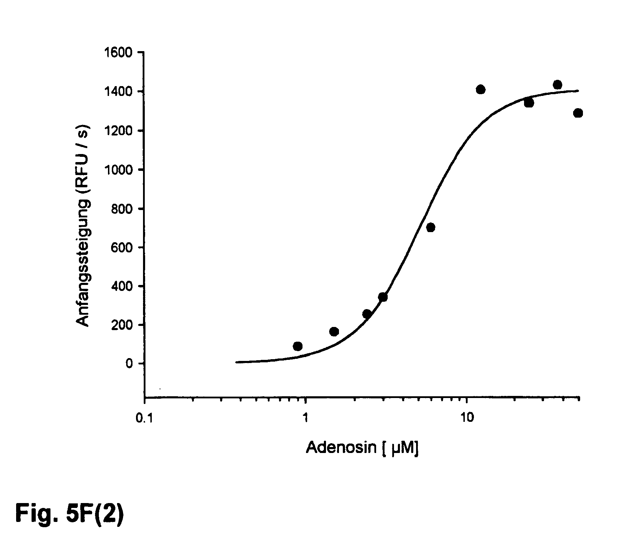

Abbildung 5F(1) zeigt, daß mit dem Verfahren auch quantitative Analysen möglich sind. Zellen, die einen endogenen Adenosin-Rezeptor (Cooper J, Hill SJ, and Alexander SP (1997). An endogenous A2B adenosine receptor coupled to cyclic AMP generation in human embryonic kidney (HEK 293) cells. Br. J. Pharmacol., 122, 546-550) und die T537M-Mutante des α3-CNG-Kanals stabil exprimieren, wurden mit unterschiedlichen Konzentrationen Adenosin stimuliert. Dargestellt ist der zeitliche Verlauf der Fluoreszenzintensität. Der Pfeil markiert den Zeitpunkt, an dem die Zellen mit Adenosin stimuliert wurden. Die Adenosin-induzierte Aktivierung des Adenosin-Rezeptors führt in der Zelle zunächst zur Aktivierung eines stimulatorischen G-Proteins und schließlich zur Aktivierung der Adenylatzyklase, die cAMP synthetisiert. Durch die Bindung von cAMP werden CNG-Kanäle geöffnet und Ca²⁺-Ionen strömen in die Zelle. Das Ausmaß und die Geschwindigkeit der Änderung der intrazellulären Ca²+-Konzentration hängt von der Adenosin-Konzentration ab. Die Adenosin-Konzentration betrug 0,9 µM (gefüllte Kreise) und 1,5 µM (offene Kreise), 3 µM (gefüllte Dreiecke), 6 µM (offene Dreiecke), 25 µM (gefüllte Vierecke) oder 37,5 µM (offene Vierecke).
Die Abbildung 5F(2) zeigt die Dosis-Wirkungsbeziehung für Adenosin. Die Geschwindigkeit, mit der sich die Fluoreszenz nach Zugabe von Adenosin ändert (Anfangsgeschwindigkeit), korreliert mit der Konzentration von Adenosin. Die Anfangsgeschwindigkeiten sind gegen die Adenosin-Konzentration aufgetragen. Die durchgezogene Linie ist nach der Hillgleichung: Anfangssteigung = a × cⁿ / (K"+c") ₙ (c = Adenosin-Konzentration; K: Konzentration, bei der die halbmaximale Anfangsgeschwindigkeit erreicht wird; n: Hillkoeffizient; a = maximale Anfangssteigung) mit folgenden Parametern berechnet worden: a =1403 RFU / s; K = 5,15 µM, n = 2,16),
Der so ermittelte K_{1/2}-Wert stimmt sehr gut mit dem in der Literatur angegebenen Wert von 5 µM überein (Peakman MC and Hill SJ. (1994) Adenosine A2B-receptor-mediated cyclic AMP accumulation in primary rat atrocytes. Br. J. Pharmacol., 111, 191-198).

### Beispiel 11

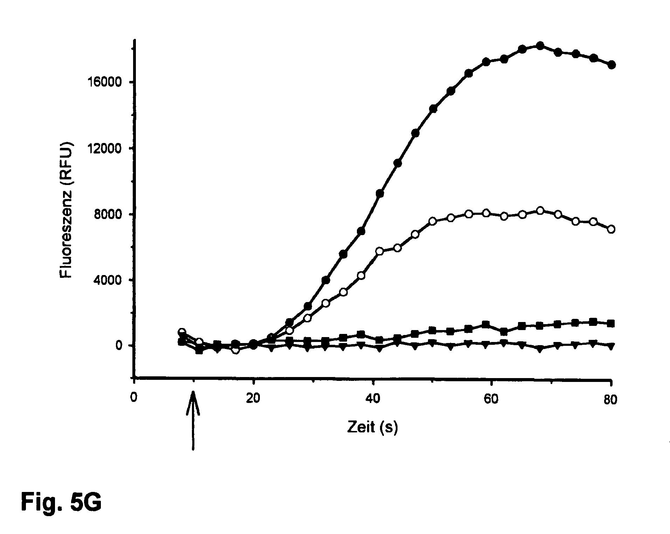

Die Abbildung 5G demonstriert die enorme Verbesserung der Empfindlichkeit des Meßsystems durch die Verwendung der modifizierten Untereinheit des α3-CNG-Kanals. Zellen, die die T537M-Mutante des α3-CNG-Kanals stabil exprimieren, wurden etwa halbmaximal mit 3 µM Adenosin (offene Kreise) oder maximal mit 25 µM Adenosin (gefüllte Kreise) stimuliert. Das Ausmaß der Änderung der intrazellulären Ca²⁺-Konzentration (Fluoreszenzsignal) wurde gegen die Zeit aufgetragen. Der Pfeil markiert den Zeitpunkt, an dem die Zellen mit Adenosin stimuliert wurden. Schon nach Stimulation mit halbmaximaler Adenosin-Konzentration ist ein deutlicher Anstieg des Fluoreszenzsignals zu beobachten. Dagegen zeigen die Zellen, die den Wildtyp-α3-CNG-Kanal stabil exprimieren, selbst nach maximaler Stimulation mit 25 µM Adenosin keine Zunahme des Fluoreszenzsignals (gefüllte Dreiecke). Zum Vergleich wurden auch Zellen stimuliert, die die T537S-Mutante des α3-CNG-Kanals stabil exprimieren. Nach maximaler Stimulation des endogenen Adenosin-Rezeptors ist nur ein sehr schwacher Anstieg des Fluoreszenzsignals zu beobachten (gefüllte Vierecke).
Durch die Verwendung der erfindungsgemässen genetisch modifizierten Untereinheiten wird die Empfindlichkeit des intrazellulären Testsystems zur Untersuchung der Wirkung von Substanzen, die den cAMP-Signalweg beeinflussen, erheblich gesteigert und erst dadurch praktisch nutzbar. Die Signale sind so groß, und das Signal-/Rausch-Verhältnis ist so gut, daß auch quantitative Analysen möglich sind.
Geeignet für die Herstellung der erfindungsgemässen Zelllinien sind (nicht nur HEK 293-Zellen) sondern prinzipiell alle eukaryontischen Zellen, die in Zellkulturen kultiviert werden können. Es können sowohl adhärent-wachsende Zellen als auch in Suspensionen wachsende Zellen verwendet werden.

## Patentansprüche

1. Genetisch modifizierte zyklisch-Nukleotid-gesteuerte Ionenkanäle (CNG-Kanäle) aus alpha3-Untereinheiten, die an der Position, die dem Threonin T537 in der alpha3-Untereinheit aus Rind gemäß SEQ ID NO:2 entspricht, so verändert sind, daß sie eine höhere Sensitivität für cAMP und/oder eine höhere Selektivität für cAMP gegenüber cGMP im Vergleich zum Wildtyp gemäß SEQ ID NO:2 aufweisen **dadurch gekennzeichnet, daß** die Aminosäure, die dem Threonin T537 in der alpha3-Untereinheit des Rindes gemäß SEQ ID NO:2 entspricht, durch Methionin ersetzt ist.

2. Genetisch modifizierte CNG-Kanäle nach Anspruch 1, **dadurch gekennzeichnet, daß** sie alpha3-Untereinheiten von CNG-Kanälen aus Rind und/oder anderen Organismen enthalten.

3. Genetisch modifizierte CNG-Kanäle aus alpha3-Untereinheiten aus Rind und/oder anderen Organismen gemäß Anspruch 1 oder Anspruch 2 dadurch gekenntzeichnet, daß sie eine homooligomere Zusammensetzung aus gleichen alpha3-Untereinheiten oder eine heterooligomere Zusammensetzung aus verschiedenen alpha3-Untereinheiten aus Rind und/oder anderen Organismen aufweisen.

4. Genetisch modifizierte CNG-Kanäle aus alpha3-Untereinheiten aus Rind und/oder anderen Organismen nach einem der Ansprüche 1 bis 3 **dadurch gekennzeichnet, daß** sie chimäre Untereinheiten enthalten.

5. Verfahren zur Herstellung von CNG-Kanälen aus Rind und/oder anderen Organismen nach einem der Ansprüche 1 bis 4 **dadurch gekennzeichnet, daß** in den Untereinheiten die Aminosäure, die dem Threonin T537 in der alpha3-Untereinheit aus Rind gemäß SEQ ID NO:2 entspricht, gegen Methionin ausgetauscht wird.

6. Verwendung der CNG-Kanäle gemäß einem der Ansprüche 1 bis 4 zur Messung der intrazellulären cAMP-Konzentration.

7. Verwendung der CNG-Kanäle gemäß einem der Ansprüche 1 bis 4 zur Bestimmung der Wirkung von Liganden, Agonisten und Antagonisten auf G-Protein-gekoppelte Rezeptoren sowie von Aktivatoren und Inhibitoren auf andere Proteine, die die intrazelluläre cAMP-Konzentration regulieren.

8. Modifizierte Nukleinsäuren **dadurch gekennzeichnet, daß** sie für alpha3-Untereinheiten der CNG-Kanäle aus Rind und/oder anderen Organismen gemäß einem der Ansprüche 1 bis 4 kodieren.

9. Expressionsvektoren **dadurch gekennzeichnet, daß** sie Nukleinsäuren gemäß Anspruch 8 enthalten.

10. Zelllinien **dadurch gekennzeichnet, daß** sie Expressionsvektoren gemäß Anspruch 9 enthalten.

11. Zelllinien nach Anspruch 10 **dadurch gekennzeichnet, daß** sie CNG-Kanäle aus Rind und/oder anderen Organismen gemäß einem der Ansprüche 1 bis 4 exprimieren.

12. Zelllinien nach einem der Ansprüche 10 oder 11 **dadurch gekennzeichnet, daß** sie Proteine, die die intrazelluläre cAMP-Konzentration regulieren, gemeinsam mit CNG-Kanälen aus Rind und/oder anderen Organismen gemäß einem der Ansprüche 1 bis 4 heterolog koexprimieren.

13. Zelllinien nach einem der Ansprüche 10 bis 12 **dadurch gekennzeichnet, daß** sie G-Protein-gekoppelte Rezeptoren, Phosphodiesterasen, Adenylatzyklasen gemeinsam mit einem CNG-Kanal aus Rind und/oder anderen Organismen gemäß einem der Ansprüche 1 bis 4 heterolog koexprimieren.

14. Verfahren zur Herstellung von Zelllinien **dadurch gekennzeichnet, daß** sie mit einem Expressionsvektor gemäß Anspruch 9 transformiert werden.

15. Verfahren nach Anspruch 14 **dadurch gekennzeichnet, daß** eukaryontische CHO-, COS- oder SF9-Zelllinien als heterologe Expressionssysteme verwendet werden.

16. Verfahren nach Anspruch 14 **dadurch gekennzeichnet, daß** Human embryonic Kidney (HEK) 293 Zelllinien als heterologe Expressionssysteme verwendet werden.

17. Verfahren zur Herstellung von Zelllinien nach einem der Ansprüche 10 - 13 **dadurch gekennzeichnet, daß** die CNG-Kanäle aus Rind und/oder anderen Organismen stabil oder transient exprimiert werden.

18. Verfahren zur Herstellung von Zelllinien nach einem der Ansprüche 12 oder 13 **dadurch gekennzeichnet, daß**
a) der CNG-Kanal aus Rind und/oder anderen Organismen stabil exprimiert wird und
b) ein Protein, das die intrazelluläre cAMP-Konzentration reguliert oder ein Protein aus der Gruppe G-Protein-gekoppelte Rezeptoren, Phosphodiesterasen oder Adenylatzyklasen transient exprimiert wird
oder
a) der CNG-Kanal aus Rind und/oder anderen Organismen transient exprimiert wird und
b) ein Protein, das die intrazelluläre cAMP-Konzentration reguliert oder ein Protein aus der Gruppe G-Protein-gekoppelte Rezeptoren, Phosphodiesterasen oder Adenylatzyklasen stabil exprimiert wird.

19. Verwendung der Zelllinien nach einem der Ansprüche 10 bis 13 zur Bestimmung der intrazellulären Ca²⁺-Konzentration.

20. Verwendung nach Anspruch 19 **dadurch gekennzeichnet, daß** die Bestimmung mit Fluoreszenz-optischen Verfahren durchgeführt wird.

21. Verwendung nach einem der Ansprüche 19 oder 20 **dadurch gekennzeichnet, daß** fluoreszierende Ca²⁺-Indikatoren verwendet werden.

22. Verwendung nach Anspruch 19 **dadurch gekennzeichnet, daß** die Bestimmung mit Lumineszenz-optischen Verfahren durchgeführt wird.

23. Verwendung nach einem der Ansprüche 19 oder 22 **dadurch gekennzeichnet, daß** luminezierende Apoaequorin oder Isoformen davon verwendet werden.

24. Verwendung gemäß einem der Ansprüche 19 bis 23, **dadurch gekennzeichnet, daß** die Bestimmung der Ca²⁺-Konzentration in Küvetten-Meßgeräten oder Ca²⁺-Imaging-Aperaturen durchgeführt wird.

25. Verwendung gemäß einem der Ansprüche 19 bis 24 **dadurch gekennzeichnet, daß** die Bestimmung der Ca²⁺-Konzentration in Fluoreszenz- bzw. Lumineszenzreadern durchgeführt wird.

26. Verwendung gemäß einem der Ansprüche 19 bis 25 **dadurch gekennzeichnet, daß** Multiwell-Platten verwendet werden.

27. Verwendung gemäß einem der Ansprüche 19 bis 26 für die Charakterisierung pharmazeutischer Wirkstoffe oder pharmakologischer Substanzen, die die intrazelluläre cAMP-Konzentration erhöhen oder verringern.

28. Verwendung gemäß einem der Ansprüche 19 bis 26 für die Charakterisierung von GPCRs, Adenylatzyklasen, Phosphodiesterasen oder anderen Proteinen, die die zelluläre cAMP-Konzentration regulieren.

29. Verwendung gemäß einem der Ansprüche 27 oder 28 **dadurch gekennzeichnet, daß** mit hohem Durchsatz (HTS) oder ultrahohem Durchsatz (UHTS) gearbeitet wird.

30. Verwendung gemäß einem der Ansprüche 19 bis 29 **dadurch gekennzeichnet, daß** die Messungen in Echtzeit durchgeführt werden.

## Claims

1. Genetically modified cyclic nucleotide-gated ion channels (CNG channels) composed of alpha3 subunits which have been modified at the position corresponding to threonine T537 in the bovine alpha3 subunit according to SEQ ID NO: 2 so that they have higher sensitivity for cAMP and/or higher selectivity for cAMP than for cGMP in comparison with the wild type according to SEQ ID NO: 2, **characterized in that** the amino acid corresponding to threonine T537 in the bovine alpha3 subunit according to SEQ ID NO: 2 has been replaced by methionine.

2. Genetically modified CNG channels according to Claim 1, **characterized in that** they comprise alpha3 subunits of CNG channels from bovine and/or other organisms.

3. Genetically modified CNG channels composed of alpha3 subunits from bovine and/or other organisms according to Claim 1 or Claim 2, **characterized in that** they have a homooligomeric composition of identical alpha3 subunits or a heterooligomeric composition of different alpha3 subunits from bovine and/or other organisms.

4. Genetically modified CNG channels composed of alpha3 subunits from bovine and/or other organisms according to any of Claims 1 to 3, **characterized in that** they comprise chimeric subunits.

5. Method for preparing CNG channels, from bovine and/or other organisms according to any of Claims 1 to 4, **characterized in that**, in the subunits the amino acid corresponding to threonine T537 in the bovine alpha3 subunit according to SEQ ID No: 2 is replaced with methionine.

6. Use of the CNG channels according to any of Claims 1 to 4 for measuring the intracellular cAMP concentration.

7. Use of the CNG channels according to any of Claims 1 to 4 for determining the action of ligands, agonists and antagonists on G protein-coupled receptors and of activators and inhibitors on other proteins regulating the intracellular cAMP concentration.

8. Modified nucleic acids, **characterized in that** they code for alpha3 subunits of the CNG channels from bovine and/or other organisms according to any of Claims 1 to 4.

9. Expression vectors, **characterized in that** they comprise nucleic acids according to Claim 8.

10. Cell lines, **characterized in that** they comprise expression vectors according to Claim 9.

11. Cell lines according to Claim 10, **characterized in that** they express CNG channels from bovine and/or other organisms according to any of Claims 1 to 4.

12. Cell lines according to either of Claims 10 and 11, **characterized in that** they heterologously coexpress proteins regulating the intracellular cAMP concentration together with CNG channels from bovine and/or other organisms according to any of Claims 1 to 4.

13. Cell lines according to any of Claims 10 to 12, **characterized in that** they heterologously coexpress G protein-coupled receptors, phosphodiesterases, adenylate cyclases together with a CNG channel from bovine and/or other organisms according to any of Claims 1 to 4.

14. Method for preparing cell lines, **characterized in that** they are transformed with an expression vector according to Claim 9.

15. Method according to Claim 14, **characterized in that** eukaryotic CHO, COS or SF9 cell lines are used as heterologous expression systems.

16. Method according to Claim 14, **characterized in that** human embryonic kidney (HEK) 293 cell lines are used as heterologous expression systems.

17. Method for preparing cell lines according to any of Claims 10-13, **characterized in that** the CNG channels from bovine and/or other organisms are expressed stably or transiently.

18. Method for preparing cell lines according to either of Claims 12 and 13, **characterized in that**
a) the CNG channel from bovine and/or other organisms is expressed stably, and
b) a protein regulating the intracellular cAMP concentration or a protein from the group of G protein-coupled receptors, phosphodiesterases or adenylate cyclases is expressed transiently,
or
a) the CNG channel from bovine and/or other organisms is expressed transiently, and
b) a protein regulating the intracellular cAMP concentration or a protein from the group of G protein-coupled receptors, phosphodiesterases or adenylate cyclases is expressed stably.

19. Use of the cell lines according to any of Claims 10 to 13 for determining the intracellular Ca²⁺ concentration.

20. Use according to Claim 19, **characterized in that** the determination is carried out using fluorescence-optical methods.

21. Use according to either of Claims 19 and 20, **characterized in that** fluorescent Ca²⁺ indicators are used.

22. Use according to Claim 19, **characterized in that** the determination is carried out using luminescence-optical methods.

23. Use according to either of Claims 19 and 22, **characterized in that** luminescent apoaequorin or isoforms thereof are used.

24. Use according to any of Claims 19 to 23, **characterized in that** the Ca²⁺ concentration is determined in cuvette measuring devices or Ca²⁺ imaging apparatuses.

25. Use according to any of Claims 19 to 24, **characterized in that** the Ca²⁺ concentration is determined in fluorescence or luminescence readers.

26. Use according to any of Claims 19 to 25, **characterized in that** multiwell plates are used.

27. Use according to any of Claims 19 to 26 for characterizing pharmaceutically active compounds or pharmacological substances, which increase or reduce the intracellular cAMP concentration.

28. Use according to any of Claims 19 to 26 for characterizing GPCRs, adenylate cyclases, phosphodiesterases or other proteins regulating the cellular cAMP concentration.

29. Use according to either of Claims 27 and 28, **characterized in that** high throughput (HTS) or ultrahigh throughput (UHTS) is employed.

30. Use according to any of Claims 19 to 29, **characterized in that** the measurements are carried out in real time.

## Revendications

1. Canaux ioniques sensibles aux nucléotides cycliques (canaux CNG) génétiquement modifiés, constitués de sous-unités alpha3, qui sur la position qui correspond à la thréonine T537 dans la sous-unité alpha3 de bovin selon SEQ ID N° 2 sont modifiés de telle sorte qu'ils présentent, par comparaison avec le type sauvage selon SEQ ID N° 2, une sensibilité plus grande pour le cAMP et/ou une sélectivité plus grande pour le cAMP par rapport au cGMP, **caractérisés en ce que** l'acide aminé qui correspond à la thréonine T537 de la sous-unité alpha3 de bovin selon SEQ ID N° 2 est remplacé par une méthionine.

2. Canaux CNG génétiquement modifiés selon la revendication 1, **caractérisés en ce qu'**ils contiennent des sous-unités alpha3 de canaux CNG de bovin et/ou d'autres organismes.

3. Canaux CNG génétiquement modifiés constitués de sous-unités alpha3 de bovin et/ou d'autres organismes selon la revendication 1 ou la revendication 2, **caractérisés en ce qu'**ils présentent une composition homo-oligomère de sous-unités alpha3 identiques ou une composition hétéro-oligomère de différentes sous-unités alpha3 de bovin et/ou d'autres organismes.

4. Canaux CNG génétiquement modifiés constitués de sous-unités alpha3 de bovin et/ou d'autres organismes selon l'une des revendications 1 à 3, **caractérisés en ce qu'**ils contiennent des sous-unités chimères.

5. Procédé de préparation de canaux CNG de bovin et/ou d'autres organismes selon l'une des revendications 1 à 4, **caractérisé en ce que**, dans les sous-unités, l'acide aminé qui correspond à la thréonine T537 de la sous-unité alpha3 de bovin selon SEQ ID N° 2 est remplacé par une méthionine.

6. Utilisation des canaux CNG selon l'une des revendications 1 à 4 pour mesurer la concentration intracellulaire du cAMP.

7. Utilisation des canaux CNG selon l'une des revendications 1 à 4 pour déterminer l'action de ligands, d'agonistes et d'antagonistes sur des récepteurs couplés à la protéine G, ainsi que d'activateurs et d'inhibiteurs, sur d'autres protéines, qui régulent la concentration intracellulaire du cAMP.

8. Acides nucléiques modifiés, **caractérisés en ce qu'**ils codent pour des sous-unités alpha3 des canaux CNG de bovin et/ou d'autres organismes selon l'une des revendications 1 à 4.

9. Vecteurs d'expression, **caractérisés en ce qu'**ils contiennent des acides nucléiques selon la revendication 8.

10. Lignées cellulaires, **caractérisées en ce qu'**elles contiennent des vecteurs d'expression selon la revendication 9.

11. Lignées cellulaires selon la revendication 10, **caractérisées en ce qu'**elles expriment des canaux CNG de bovin et/ou d'autres organismes selon l'une des revendications 1 à 4.

12. Lignées cellulaires selon l'une des revendications 10 ou 11, **caractérisées en ce qu'**elles co-expriment d'une manière hétérologue des protéines qui régulent la concentration intracellulaire du cAMP, en commun avec des canaux CNG de bovin et/ou d'autres organismes selon l'une des revendications 1 à 4.

13. Lignées cellulaires selon l'une des revendications 10 à 12, **caractérisées en ce qu'**elles co-expriment d'une manière hétérologue des récepteurs couplés à la protéine G, des phosphodiestérases, des adénylate-cyclases, en commun avec un canal CNG de bovin et/ou d'autres organismes selon l'une des revendications 1 à 4.

14. Procédé de fabrication de lignées cellulaires, **caractérisé en ce qu'**elles sont transformées avec un vecteur d'expression selon la revendication 9.

15. Procédé selon la revendication 14, **caractérisé en ce qu'**on utilise en tant que systèmes d'expression hétérologues les lignées cellulaires eucaryotes CHO, COS ou SF9.

16. Procédé selon la revendication 14, **caractérisé en ce qu'**on utilise en tant que systèmes d'expression hétérologues des lignes cellulaires de rein embryonnaire humain (HEK) 293.

17. Procédé de fabrication de lignées cellulaires selon l'une des revendications 10 à 13, **caractérisé en ce que** les canaux CNG de bovin et/ou d'autres organismes sont exprimés de manière stable ou transitoire.

18. Procédé de fabrication de lignées cellulaires selon l'une des revendications 12 ou 13, **caractérisé en ce que**
a) le canal CNG de bovin et/ou d'autres organismes est exprimé de manière stable, et
b) une protéine qui régule la concentration intracellulaire du cAMP, ou une protéine du groupe des récepteurs couplés à la protéine G, des phosphodiestérases ou des adénylate-cyclases, est exprimée de manière transitoire,
ou
a) le canal CNG de bovin et/ou d'autres organismes est exprimé de manière transitoire, et
b) une protéine qui régule la concentration intracellulaire du cAMP ou une protéine du groupe des récepteurs couplés à la protéine G, des phosphodiestérases ou des adénylate-cyclases, est exprimée de manière stable.

19. Utilisation des lignées cellulaires selon l'une des revendications 10 à 13 pour déterminer la concentration intracellulaire du Ca²⁺.

20. Utilisation selon la revendication 19, **caractérisée en ce que** la détermination est mise en oeuvre par un procédé de fluorescence optique.

21. Utilisation selon l'une des revendications 19 ou 20, **caractérisée en ce qu'**on utilise des indicateurs fluorescents de Ca²⁺.

22. Utilisation selon la revendication 19, **caractérisée en ce que** la détermination est mise en oeuvre avec des procédés de luminescente optique.

23. Utilisation selon l'une des revendications 19 ou 22, **caractérisée en ce qu'**on utilise une apoaequorine luminescente ou des isoformes de cette dernière.

24. Utilisation selon l'une des revendications 19 à 23, **caractérisée en ce que** la détermination de la concentration du Ca²⁺ est mise en oeuvre dans des appareils de mesure à cuvette ou dans des appareils d'imagerie du Ca²⁺.

25. Utilisation selon l'une des revendications 19 à 24, **caractérisée en ce que** la détermination de la concentration du Ca²⁺ est réalisée dans des lecteurs de fluorescence ou de luminescence.

26. Utilisation selon l'une des revendications 19 à 25, **caractérisée en ce qu'**on utilise des plaques multipuits.

27. Utilisation selon l'une des revendications 19 à 26 pour la caractérisation de principes actifs pharmaceutiques ou de substances pharmacologiques qui augmentent ou diminuent la concentration intracellulaire du cAMP.

28. Utilisation selon l'une des revendications 19 à 26 pour la caractérisation de GPCR, d'adénylate-cyclases, de phosphodiestérases ou d'autres protéines qui régulent la concentration cellulaire du cAMP.

29. Utilisation selon l'une des revendications 27 ou 28, **caractérisée en ce qu'**elle fonctionne à haut débit (HTS) ou à ultra-haut débit (UHTS).

30. Utilisation selon l'une des revendications 19 à 29, **caractérisée en ce que** les mesures sont effectuées en temps réel.
